# EUROPEAN PATENT APPLICATION

(11) **EP 1 834 671 A2**
(43) Date of publication of application: **19.09.2007**
(21) Application number: 06076491.7
(22) Date of filing: 08.07.1993
(51) Int. Cl.: A61P 37/00, A61K 39/395, C07K 16/28, C12N 5/12

(54) **A method for generation of antibodies to cell surface molecules**

(30) Priority: 09.07.1992 US 910222; 09.02.1993 US 15147; 28.05.1993 US 70158
(62) Divisional of application: 99104709.3
(71) Applicant: Novartis Vaccines and Diagnostics, Inc., Emeryville, CA 94608 (US)
(72) Inventor: De Boer, Mark, 1261 BT Blaricum (NL); Conroy, Leah B., Pacifica, CA 94044 (US)
(74) Representative: Marshall, Cameron John

(57) **Abstract**

The present invention relates to a method of generating antibodies directed against cell surface antigens and it uses recombinant insect cells as immunogens. The insect cells have been transfected with coding regions for a molecule containing a cell surface antigen and these antigens are expressed on the surface of the insect cells. Host animals are immunized with these transfected insect cells to generate antibodies directed against the cell surface antigen. Antibody-producing cells from the host animal are used to generate monoclonal antibody-producing hybridoma cells. Sera and hybridoma supernatants can be tested for the presence of antibodies against the surface antigen, using the transfected cells in a screening assay. Specific antibodies that have been generated by the present method are anti-B7 and anti-CD40 antibodies and they can be used to prevent or treat an antibody-mediated or immune system disease in a patient. The anti-B7 antibodies can be used to cause T cell anergy, treat allograft transplant rejection, treat graft versus host disease, and prevent or treat rheumatoid arthritis. An immunosuppressive agent may be co-administered with the antibody. The anti-CD40 antibodies which bind to the CD40 antigen can be used to prevent the growth or differentiation of the B cell. Monoclonal antibodies useful in these methods, and epitopes immunoreactive with such monoclonal antibodies are also presented.

## Description

### Field of the invention

The present invention relates to a method of generating antibodies directed against membrane-associated antigen molecules. More specifically, the present invention describes methods of using these membrane-associated antigens for the immunization of a host animal and for screening antibodies isolated from the host animal. Also, this invention relates to novel methods of treating diseases of the immune system. In particular, this invention relates to methods of preventing or treating (1) transplant rejection, graft versus host disease, and other immunological conditions arising from the recognition of specific antigens as foreign, and (2) antibody-mediated diseases such as IgE-mediated disease (allergies) and autoimmune diseases including sytematic lupus erythematosus (SLE), primary biliary cirrhosis (PBC), and idiopathic thrombocytopenic purpura (ITP).

### Background of the invention

### I. Producing Monoclonal Antibodies.

Monoclonal antibodies have proven to be powerful tools in immunological research. In general, monoclonal antibodies can be produced using impure antigens as immunogens, provided that there is available a screening assay which distinguishes antibodies directed against the antigen of interest from antibodies directed against other antigens present in the immunogenic composition. When the antigen of interest is a cell surface molecule, it is desirable to use cells or membrane fractions containing the molecule of interest as immunogens in order to preserve the conformational constraints provided by a membrane environment.

Immunizing mice with whole cells usually yields a strong immune response which generates antibodies to a large number of different molecules. This broad immune response precludes the use of the immunogen cells in subsequent screening for specific antibody production by hybridoma clones derived from the mouse spleen or lymphocyte cells.

Furthermore, when the antigen of interest is expressed at low density, it is likely that the frequency of mouse B cells specific for the antigen will be relatively low. This low frequency necessitates the screening of large numbers of hybridoma clones to identify a clone which produces antibodies directed against the antigen of interest.

Syngeneic murine fibroblasts expressing human cell surface antigens have been used to immunize mice for specific antibody production (DiSanto *et al.,* 1991). When injected into the appropriate mouse strain, the background antigen proteins present on the fibroblasts should not be immunogenic, so that the immune response should be focused on the xenogeneic recombinant protein. However, this approach requires the construction of specific recombinant cells for each species or strain in which antibody production is desired.

### II. Graft Rejection and the CD28-B7 System.

Current strategies for the prevention of graft rejection after transplantation are based on the use of broad acting immunosuppressive agents such as cyclosporin A (CsA), FK506 and corticosteroids. These drugs must often be taken over long periods of time and therefore increase the risk of serious infections, nephrotoxicity, and cancer. In addition, not all patients can tolerate high doses of these immunosuppressive agents, often resulting in graft rejection or graft-versus-host disease (GVHD). optimal prevention of graft rejection should be based on the induction of specific tolerance to the donor tissue. Thus, the ideal drug for prevention of transplant rejection should induce clonal unresponsiveness, or anergy, of donor-reactive T cells, without the need for long-term immunosuppression. Anergy is thought to be the result of intercellular signaling after interaction between the T-cell receptor (TCR) and the peptide-presenting major histocompatibility complex (MHC) antigen in the absence of a "costimulatory" signal. Mueller, D.L. et al., Annu. Rev. Immunol. (1989) 7:445. This costimulatory signal is normally provided by the cell surface of antigen-presenting cells (APCs). Hawrylowicz, C.M. et al., J. Immunol. (1988) 141:4083; and Springer, T.A. et al., Annu. Rev. Immunol. (1987) 5:223.

T cells play an important role during the normal in vivo immune response. They are involved in cytotoxicity, delayed type hypersensitivity, and T cell-dependent antibody production by B cells. Furthermore, T cells can produce a wide variety of lymphokines such as interleukin-2 (IL-2), tumor necrosis factor alpha (TNF-α), lymphotoxin, gamma interferon (IFN-γ), and granulocyte macrophage colony stimulating factor (GM-CSF).

The activation of T cells is the result of ligand-receptor interactions. Under physiological conditions, the TCR/CD3 complex binds to antigenic peptides presented by the MHC molecules of APCs. The TCR/CD3 complex plays two roles in antigen-induced activation. First, it recognizes a specific antigen in the context of an antigen-presenting MHC molecule. Then, the recognition event is transmitted through the plasma membrane by a signalling mechanism. However, binding of antigen to the TCR alone is not sufficient for maximum T cell activation. A number of other accessory molecules on the surface of the T cell are known to play important roles in adhesion or signalling or both. For instance, the CD2 molecule on T cells can bind to LFA-3 on APCs, but it has also been shown that binding of antibodies to CD2 can augment the signals provided by the TCR/CD3 complex. Other ligand pairs involved in T cell activation are LFA-1/ICAM-1, CD4/MHC-class II antigen, VLA-4/VCAM, and, most importantly, CD28/B7.

The likely candidate for the costimulatory signal that determines whether TCR-stimulation leads to full T cell activation or to T cell anergy is that generated by interaction of CD28 on the T cells with B7 on APCs. It has been demonstrated in vitro that cross-linking of the CD28 molecule can rescue T cells from becoming anergic. Harding, F.A. et al., Nature (1992) 356:607. CD28 is a homodimeric transmembrane glycoprotein with an apparent molecular mass of 44 kDa and is a member of the immunoglobulin superfamily (Aruffo, A. & Seed, B. PNAS (USA) (1987) 84:8573). The CD28 molecule is expressed on approximately 95% of CD4-positive T cells and 50% of CD8-positive T cells. Costimulation of T cells with monoclonal antibody to the TCR/CD3 complex and CD28 results in greatly enhanced T cell activation. Thompson, C.B. et al., PNAS (USA) (1989) 86:1333-1337; June, C.H. et al., J, Immunol. (1989) 143:153-161; and Lindsten, T. et al., Science (1989) 244:339-343. This effect apparently involves stabilization of mRNA for several lymphokines, including IL-2, resulting in a greatly enhanced production of these lymphokines. June, C.H. et al., supra; and Lindsten, T. et al., supra. Furthermore, a CD28-responsive element has been demonstrated in the enhancer of the IL-2 gene, suggesting that there is also regulation at the transcriptional level. Fraser, J.D. et al., Science (1991) 251:313 and Verwey, C.L. et al., J. Biol. Chem. (1991) 266:14179-14182. Certain models of T cell activation mediated by CD28 have been reported to be relatively resistant to inhibition with CsA. June, C.H. et al., Mol. Cell. Biol. (1987) 7:4472-4481.

B7 is a monomeric transmembrane glycoprotein with an apparent molecular mass of 45-65 kDa and is, like CD28, a member of the immunoglobulin superfamily. Freeman, G.J. et al., J. Immunol. (1989) 143:2714-2722. Moreover, B7-expressing CHO cells are able to synergize with TCR stimulation, resulting in IL-2 secretion and T cell proliferation. Linsley, P.S. et al., J. Exp. Med. (1991) 137:721-730; and Gimmi, C.D. et al., PNAS (USA) (1991) 88:6575. B7 also binds to a recombinant fusion protein of the CTLA-4 molecule. Linsley, P.S. et al., J. Exp. Med. (1991) 174:561-569. CTLA-4, too, is a member of the immunoglobulin superfamily, and the cytoplasmic regions of CTLA-4 and CD28 show significant homology. Harper, K. et al., J. Immunol. (1991) 147:1037. The B7 molecule is expressed on activated B cells (Freeman, G.J. et al., supra), monocytes stimulated with IFN-γ (Freedman, A.S. et al., Cell. Immunol. (1991) 137:429-437), and isolated peripheral blood dendritic cells (Young, J.W. et al., J. Clin. Invest. (1992) 90:229-237). Immunohistochemical studies have shown that the B7 molecule is also constitutively expressed *in vivo* on dendritic cells in both lymphoid and non-lymphoid tissue. Vandenberghe, P. et al., "International Immunology" (1993).

*In vivo,* the B7 antigen is involved in T cell activation during transplant rejection. Lenschow and co-workers have used a soluble fusion protein of human CTLA-4 and the immunoglobulin G1 Fc region (CTLA4Ig), which strongly binds to both mouse and human B7, to prevent rejection of human pancreatic islets after transplantation in mice (Lenschow, D.J. et al., Science (1992) 257:789-792). Here CTLA4Ig blocks rejection of a xenoantigen (an antigen foreign to the species from which the T cell is derived).

Molecules that interfere with the interaction between the B7 and CD28 antigens are known in the art. The soluble CTLA4-Ig fusion protein is known to partially block this interaction. Linsley, P.S. et al., J. Exp. Med. (1991) 74:561. Anti-CD28 antibodies are also known to block this interaction. Furthermore, anti-B7 antibodies are known in the art. Yokochi, T. et al., J. Immunol. (1982) 128:823; Freedman, A.S. et al. J. Immunol. (1987) 139:3260; Valle, A. et al. Immunol (1990) 69:531.

### III. B-Cell Activation and the CD40 Antigen-Ligand System.

B cells play an important role during the normal *in vivo* immune response. A foreign antigen will bind to surface immunoglobulins on specific B cells, triggering a chain of events including endocytosis, processing, presentation of processed peptides on MHC-class II molecules, and up-regulation of the B7 antigen on the B-cell surface. A specific T cell then binds to the B cell via T-cell receptor (TCR) recognition of processed antigen presented on the MHC-class II molecule. Stimulation through the TCR begins to activate the T cell and initiates T-cell cytokine production. Interaction between the CD28 antigen on T cells and the B7 antigen on B cells can provide a second signal further activating the T cell, resulting in high level cytokine secretion. Additionally, the CD40 ligand, which is not expressed on resting human T cells, is up-regulated on the T-cell surface when the above-mentioned signals are received. The B cell is then stimulated by the CD40 ligand through the CD40 antigen on the B-cell surface, and also by soluble cytokines, causing the B cell to mature into a plasma cell secreting high levels of soluble immunoglobulin.

A few years ago, Zubler et al., J. Immunol. (1985) 134:3662, observed that a mutant subclone of the mouse thymoma EL-4 line, known as EL4B5, could strongly stimulate B cells of both murine and human origin to proliferate and differentiate into immunoglobulin-secreting plasma cells *in vitro.* This activation was found to be antigen-independent and not MHC restricted. For optimal stimulation of human B cells, the presence of supernatant from activated human T cells was needed, but a B-cell response also occurred when EL4B5 cells were preactivated with phorbol-12-myristate 13-acetate (PMA) or IL-1. Zubler et al., Immunological Reviews (1987) 99:281; and Zhang et al., J. Immunol. (1990) 144:2955. B-cell activation in this culture system is efficient -- limiting dilution experiments have shown that the majority of human B cells can be activated to proliferate and differentiate into antibody-secreting cells. Wen et al. Eur. J. Immunol. (1987) 17:887.

The mechanism by which these mutant EL-4 cells activate both murine and human B cells has not been elucidated previously. It is, however, clear that cell-cell contact is required for EL4B5-induced B-cell activation. First, B cells do not proliferate in the presence of supernatant from PMA-stimulated EL4B5 cells. Zubler et al. (1985) supra. Second, B cells do not proliferate when they are separated from PMA-treated EL4B5 cells by a semipermeable filter membrane. Zhang et al., supra. Antibodies against mouse LFA-1, human LFA-1 or human LFA-3 and antibodies against mouse or human MHC class II molecules do not inhibit EL4B5-induced proliferation of human or murine B cells. Zubler et al. (1987) and Zhang et al., supra.

The CD40 antigen is a glycoprotein expressed on the cell surface of B cells. During B-cell diferentiation the molecule is first expressed on pre-B cells and then disappears from the cell surface when the B cell becomes a plasma cell. Crosslinking of the CD40 molecules with anti-CD40 antibodies mediates a variety of effects on B cells. The CD40 antigen is known to be related to the human nerve growth factor (NGF) receptor and tumor necrosis factor-alpha (TNF-α) receptor, suggesting that CD40 is a receptor for a ligand with important functions in B-cell activation.

A ligand for CD40 has been identified on the cell surface of activated T cells. Fenslow et al., J. Immunol. (1992) 149:655; Lane et al., Eur. J. Immunol. (1992) 22:2573; Noelle et al., Proc. Natl. Acad. Sci. (USA) (1992) 89:6550. cDNA cloning of the CD40 ligand revealed a molecule with characteristics of a type-II transmembrane glycoprotein with homology to TNF-α. Armitage et al., Nature (1992) 357:80 and Spriggs et al., J. Exp. Med. (1992) 176:1543. The extracellular domain of the CD40 ligand contains two arginine residues proximal to the transmembrane region, providing a potential proteolytic cleavage site that could give rise to a soluble form of the ligand. Expression of recombinant CD40 ligand has demonstrated that this molecule can stimulate the proliferation of purified B cells and, in combination with IL-4, mediate the secretion of IgE. Armitage et al. and Spriggs et al., supra. It has been reported that abnormalities in the gene for the CD40 ligand, resulting in the absence of a functional molecule on activated T cells, is responsible for the occurrence of X-linked hyper-IgM syndrome, a rare disorder characterized by the inability of these patients to produce normal levels of antibody isotypes other than IgM. Allen et al., Science (1993) 259:990; and Korthäuer et al., Nature (1993) 361:539.

All anti-CD40 antibodies known in the art have a stimulatory effect on human B cells. Cross-linking of the CD40 molecule on the B-cell surface using known anti-CD40 antibodies mediates a variety of effects on B cells. Anti-CD40 monoclonal antibodies (mAbs) can induce intercellular adhesion, proliferation and, in combination with certain cytokines, maturation to antibody secreting cells. For example, known anti-CD40 mAbs have been shown to mimic the effects of T helper cells in B-cell activation. When presented on adherent cells expressing FcγRII, these antibodies induce B-cell proliferation. J. Banchereau et al., Science (1989) 251:70. Moreover, the known anti-CD40 mAbs can replace the T helper signal for secretion of IgM, IgG and IgE in the presence of IL-4. H. Gascan et al., J. Immunol. (1991) 147:8. Furthermore, known anti-CD40 mAbs can prevent programmed cell death (apoptosis) of B cells isolated from lymph nodes.

The present invention provides a method for generating monoclonal antibody-producing cells, where the antibodies have binding specificity for a specific cell-surface molecule; this method overcomes the limitations of the above-described methods. The invention also provides methods for the use of anti-B7 antibodies or molecules that bind to the B7 antigen in conjunction with other immunosuppressive agents to cause T cell anergy and thereby prevent or treat graft rejection or GVHD. Furthermore, the present invention also provides new anti-CD40 antibodies that are (1) capable of inhibiting the B-cell response and (2) can be used to prevent or treat antibody-mediated disease.

### Summary of the invention

The present invention provides a method of generating immortalized cell lines capable of producing a monoclonal antibody specifically reactive against a selected membrane-associated antigen. In the method, the membrane-associated antigen is produced on the cell surface of insect cells, typically via recombinant expression of antigen coding sequences using baculoviral vectors. These insect cells are injected into a host animal. Cells capable of producing antibodies directed against the membrane-associated antigen are recovered; such cells are immortalized for growth in cell culture. The immortalized cells are screened for production of antibodies specific to the membrane-associated antigen. The screening is typically accomplished using a binding assay employing non-insect cells having the membrane-associated antigen on the surface. Immortalized cells producing antibodies which bind to the membrane-associated antigen on the surface of said non-insect cells are selected.

In one embodiment, DNA sequences encoding the membrane-associated antigen are inserted operatively into a baculoviral vector suitable for expression of the antigen in selected insect cells. The vector carrying the DNA sequences encoding the antigen are introduced into insect cells, for example, by transfection with the vector. Insect cells transformed with the vector-which produce the membrane-associated antigen on the cell surface are selected.

One method of isolating target DNA sequences, encoding the membrane-associated antigen of interest, is using the polymerase chain reaction employing either DNA or RNA templates containing the target DNA sequences. Insect cells useful in the practice of the present invention can be obtained from *Spodoptera frugiperda.* Commonly used host animal cells, capable of producing antibody molecules directed against the membrane associated antigen, are splenic cells or lymphocytes; immortalization of the host animal cells is accomplished by standard procedures. In one embodiment of the present invention, the host animal is a mouse.

In the above described method, a number of non-insect cells, having the membrane-associated antigen on the surface, can be employed, including human cells. Such non-insect cells can be primary or cultured cells. In one embodiment, the non-insect cells can be cultured human lymphocytes, especially transformed B cells.

In the method of the present invention, useful membrane-associated antigens include the following: human cell surface proteins; antigenic marker proteins for a peripheral blood mononuclear cells, for example, CD40 or B7; and viral antigenic proteins present on the surface of a human cell.

The present invention also describes a method of producing sera containing antibodies specific against a selected membrane-associated antigen, where the membrane-associated antigen is present on the cell surface of insect cells, the cells are injected into a host animal, and sera from the animal are screened for the presence of antibodies specific to the membrane-associated antigen. such screening typically employs a binding assay using non-insect cells having the membrane-associated antigen on the cell surface.

Sera and hybridoma supernatants can also be tested for the presence of antibodies against a selected membrane-associated antigen using, in a screening assay, recombinant insect cells expressing the antigen.

The current invention is also based on the discovery that the coadministration of a molecule that binds to the B7 antigen (such as the anti-B7 monoclonal antibody made by the above-described method) and an immunosuppressive agent to a patient can induce long-lasting T cell anergy against an alloantigen (an antigen native to the same species as the T cell).

Accordingly, this invention provides a method for preventing transplant rejection in a patient, the method comprising administering to a patient in need of such treatment therapeutically effective amounts of (1) a molecule that binds to the B7 antigen and (2) an immunosuppressive agent such as cyclosporin A, FK506, or a corticosteroid, in a pharmaceutically acceptable excipient. In a preferred embodiment of this invention, the molecule that binds to the B7 antigen is an anti-B7 antibody.

Additionally, this invention provides a method for preventing or treating graft versus host disease (GVHD) in a patient, the method comprising administering to a patient in need of such treatment therapeutically effective amounts of (1) a molecule that binds to the B7 antigen and (2) an immunosuppressive agent such as cyclosporin A, FK506, or a corticosteroid, in a pharmaceutically acceptable excipient. In a preferred embodiment of this invention, the molecule that binds to the B7 antigen is an anti-B7 antibody.

Also, this invention provides a method for preventing or treating rheumatoid arthritis in a patient, the method comprising administering to a patient in need of such treatment therapeutically effective amounts of (1) a molecule that binds to the B7 antigen and (2) an immunosuppressive agent such as cyclosporin A, FK506, or a corticosteroid, in a pharmaceutically acceptable excipient. in a preferred embodiment of this invention, the molecule that binds to the B7 antigen is an anti-B7 antibody.

In more preferred embodiments of the above methods, the anti-B7 antibody is a monoclonal antibody, most preferably the B7-24 monoclonal antibody, made as described herein.

The invention is further based on the discovery of anti-CD40 antibodies, made by the above-described method, that do not stimulate the growth and differentiation of human B cells. In contrast, these antibodies can inhibit human B-cell responses at relatively low concentrations. Accordingly, these antibodies can be used to prevent or treat diseases or conditions that are mediated by antibodies produced by the human B-cell response. These antibodies also recognize novel epitopes on the CD40 antigen useful in modulating the B-cell response.

Accordingly, this invention provides a monoclonal antibody capable of binding to a human CD40 antigen located on the surface of a human B cell, wherein the binding of the antibody to the CD40 antigen prevents the growth or differentiation of the B cell.

Additionally, this invention provides a method for preventing or treating an antibody-mediated disease in a patient, the method comprising administering to a patient in need of such treatment a therapeutically effective amount of a monoclonal antibody capable of binding to a human CD40 antigen located on the surface of a human B cell, wherein the binding of the antibody to the CD40 antigen prevents the growth or differentiation of the B cell, in a pharmaceutically acceptable excipient.

Also, this invention provides a method for preventing or treating an IgE-mediated disease such as an allergy in a patient, the method comprising administering to a patient in need of such treatment a therapeutically effective amount of a monoclonal antibody capable of binding to a human CD40 antigen located on the surface of a human B cell, wherein the binding of the antibody to the CD40 antigen prevents the growth or differentiation of the B cell, in a pharmaceutically acceptable excipient.

Furthermore, this invention provides a method for preventing or treating an antibody-mediated autoimmune disease in a patient, the method comprising administering to a patient in need of such treatment a therapeutically effective amount of a monoclonal antibody capable of binding to a human CD40 antigen located on the surface of a human B cell, wherein the binding of the antibody to the CD40 antigen prevents the growth or differentiation of the B cell, in a pharmaceutically acceptable excipient. Particular autoimmune diseases contemplated for treatment by this method include sytematic lupus erythematosus (SLE), primary biliary cirrhosis (PBC), and idiopathic thrombocytopenic purpura (ITP).

Additionnally, this invention provides a CD40 antigen epitope capable of competing with the binding of a CD40 antigen to an anti-CD40 monoclonal antibody wherein the binding of that antibody to a human CD40 antigen located on the surface of a human B cell prevents the growth or differentiation of the B cell.

In more preferred embodiments of the above objects, the anti-CD40 monoclonal antibody is made by the above-described methods, and is either 5D12, 3A8 or 3C6.

### Brief Description of the Figures

Figure 1A shows a schematic representation of the baculoviral transfer vector pAcC8 and the sequence of the multiple cloning site. Figure 1B shows a schematic representation of the generation of Sf9 cells which express human CD40 or B7 antigen according to the method of the present invention.
Figure 2 shows the sequences of polymerase chain reaction primers used in the preparation of coding regions for human CD40 and human B7 antigens. These primers were constructed on the basis of the published complete DNA coding sequences for antigens B7 and CD40 (Freeman *et al.,* 1989; Stamenkovic et al., 1989).
Figure 3 shows the results of ELISA assays examining the reaction of anti-(B7) monoclonal antibody BB-1 with Sf9 cells infected with AcB7 virus and with Sf9 cells expressing human CD26.
Figure 4 the results of ELISA assays examining the reaction of anti-(CD40) monoclonal antibody S2C6 with Sf9 cells expressing CD40 and Sf9 cells expressing B7.
Figures 5A-C show the results of the fluorescent cell staining of EBV-transformed B cell line ARC cells expressing CD40 or B7.
Figures 6A-F show the results of competition assays using fluorescent cell staining of EBV-transformed B cell line ARC cells expressing CD40 and B7, as well as soluble CD40 and B7 antigens.
Figure 7 shows the inhibition of anti-CD3-induced, B7-mediated proliferation of T cells by Fab fragments of anti-B7 Mab B7-24.
Figure 8 shows the inhibition of anti-CD3-induced, B7-mediated proliferation of T cells by anti-B7 Mabs B7-24 (squares) or BB-1 (triangles).
Figure 9 shows the effect of blocking B7/CD28 interaction using (A) MAb B7-24 or (B) NAb BB-1 during 3-day primary MLC (closed symbols), and 3-day secondary MLC (open symbols).
Figure 10 shows the inhibition of anti-CD3-induced, B7-mediated proliferation of T cells by CsA. Data are the mean ± S.D. of 3 individual experiments using T cells of different donors.
Figure 11 shows the synergistic effects of anti-B7 Mab B7-24 and CsA in blocking allo-antigen-induced CTL activity during secondary MLC. CTL activity of the T cells was analyzed after restimulation for 3 days with the EBV-transformed B cell line ARC in the presence of medium alone; 400 µg/ml CsA; 10 µg/ml Mab B7-24; or 400 µg/ml CsA and 10 µg/ml Mab B7-24.
Figure 12A compares the ability of three new and one old anti-CD40 mAbs to co-stimulate anti-IgM induced human B-cell proliferation. Figure 12B repeats the experiment of Figure 5A in the presence of recombinant interleukin-2 (rIL-2).
Figure 13 shows the ability of three new anti-CD40 mAbs to inhibit human B-cell proliferation induced by costimulation with immobilized anti-IgM and anti-CD40 mAb 52C6.
Figure 14 shows the effect of three new anti-CD40 mAbs on EL4B5-induced human B-cell proliferation.
Figure 15 shows the effect of soluble CD40 (hCD40.µ) on EL4B5-induced human B-cell proliferation.
Figures 16A and 16B show the effect of one new anti-CD40 mAb 5D12 on human T-cell induced immunoglobulin production by human B cells.

### Detailed Description of the Invention

The invention described herein draws on previously published work and pending patent applications. By way of example, such work consists of scientific papers, patents or pending patent applications. All of these publications and applications, cited previously or below are hereby incorporated by reference.

### I. Definitions:

As used herein, the term "membrane-associated antigen", "cell surface molecule" and "cell surface antigen" all refer to a protein, polypeptide or peptide, where at least one antigenic portion of the protein, polypeptide or peptide is exposed on a surface of a biological membrane and which may have one or more of the following moieties covalently attached: one or more simple or complex sugar moieties (as in a glycoprotein), lipid moieties (as in a lipoprotein), a combination of lipid and sugar moieties, or other post-translational modifications. "Proteins" are typically long chains of amino acid based polymers ("polypeptides"). Proteins may be composed of one, two or more polypeptide chains and may further contain some other type of substance in association with the polypeptide chain(s), such as carbohydrates. The size of proteins covers a rather wide range from (an arbitrary figure of) 5,000 to several hundred thousand g/mole. The 5,000 figure corresponds to the presence of roughly 40-45 amino acids. Proteins smaller than about 5,000 g/mole are typically referred to as polypeptides or simply peptides (Bohinski).

As used herein, the term "antibody" refers to polyclonal antibodies, monoclonal antibodies, humanized antibodies, single-chain antibodies, and fragments thereof such as F_{ab}, F_{(ab')2}, Fᵥ, and other fragments which retain the antigen binding function of the parent antibody.

As used herein, the term "monoclonal antibody" refers to an antibody composition having a homogeneous antibody population. The term is not limited regarding the species or source of the antibody, nor is it intended to be limited by the manner in which it is made. The term encompasses whole immunoglobulins as well as fragments such as F_{ab}, F_{(ab')2}, Fᵥ, and others which retain the antigen binding function of the antibody. Monoclonal antibodies of any mammalian species can be used in this invention. In practice, however, the antibodies will typically be of rat or murine origin because of the availability of rat or murine cell lines for use in making the required hybrid cell lines or hybridomas to produce monoclonal antibodies.

As used herein, the term "humanized antibodies" means that at least a portion of the framework regions of an immunoglobulin are derived from human immunoglobulin sequences.

As used herein, the term "single chain antibodies" refer to antibodies prepared by determining the binding domains (both heavy and light chains) of a binding antibody, and supplying a linking moiety which permits preservation of the binding function. This forms, in essence, a radically abbreviated antibody, having only that part of the variable domain necessary for binding to the antigen. Determination and construction of single chain antibodies are described in U.S. Patent No. 4,946,778 to Ladner et al.

As used herein, the term "molecule which binds to the B7 antigen" means a molecule which is capable of forming a complex with the B7 antigen in an environment wherein other substances in the same environment are not complexed to the B7 antigen. The complex is formed in a manner that blocks the normal signal transduction pathway of B7 through the CD28 or CTLA4 antigen. Molecules which bind to the B7 antigen include CD28, CTLA4, CTLA4Ig and anti-B7 antibodies.

The term "CD40 antigen epitope" as used herein refers to a molecule which is capable of immunoreactivity with the anti-CD40 monoclonal antibodies of this invention, excluding the CD40 antigen itself. CD40 antigen epitopes may comprise proteins, protein fragments, peptides, carbohydrates, lipids, and other molecules, but for the purposes of the present invention are most commonly proteins, short oligopeptides, oligopeptide mimics (i.e., organic compounds which mimic the antibody binding properties of the CD40 antigen), or combinations thereof. Suitable oligopeptide mimics are described, inter alia, in PCT application US91/04282.

### II. Generating Antibodies to Membrane-Associated Antigen Molecules

This section describes a method for generating and selecting antibodies to a cell surface molecule, using transfected insect cells as immunogen. The transfected insect cells of the present invention may also be used in screening assays.

According to an one embodiment, the invention includes a method for producing polyclonal antibodies to a cell surface antigen. The method involves the following steps: the immunization step, which includes (i) selecting and isolating a nucleic acid coding sequence which encodes the antigen of interest, (ii) inserting the coding sequence into a baculoviral expression vector so as to obtain efficient expression of the coding sequence, (iii) transfecting the expression vector into an insect cell line to obtain recombinant insect cells expressing the selected antigen, and (iv) immunizing a host animal with the insect cells expressing the membrane-associated antigen.

After immunization, the serum of the host animal is screened against cells, other than the insect cells, expressing the antigen of interest. Alternatively, membrane fractions containing the antigen of interest, or in some cases, purified recombinantly-produced antigens themselves can be used to screen the serum. Typically, (a) pre-bleed serum, (b) the serum of a host animal immunized with insect cells not expressing the antigen of interest, and (c) the serum of the host animal immunized with the recombinant insect cells are screened. The presence of antibodies specifically directed against the antigen of interest is indicated by negative reactions with sera (a) and (b), and positive reactions with serum (c).

According to another embodiment, the invention includes a method for the generation of hybridomas which produce monoclonal antibodies to a cell surface protein. The method involves the steps (i) to (iv) described above. After immunization of the host animal with the recombinant insect cells, antibody-producing cells are isolated from the animal. In a preferred embodiment of the invention, such antibody-producing cells are used to generate hybridoma cells, which are cloned and used for the production of monoclonal antibodies. Supernatants from such hybridoma cells are screened for specific antibody production, for example, using a cell-based screening assay described below.

### A. Isolating Coding Sequences for Membrane Molecules.

The nucleic acid coding sequence for a selected membrane-associated antigen can be isolated based on known amino acid and/or DNA coding sequences for the protein component of the antigen. The coding sequence can be isolated from biological sources by standard procedures (Ausubel, *et al.*; Maniatis, *et al.*; Sambrook, *et al.*) (e.g., hybridization, differential hybridization, cloning and plaque screening, etc.). Alternatively, synthetic oligonucleotide sequences encoding the antigen of interest can be prepared using commercially available automated oligonucleotide synthesizers or may be purchased, for example, from Synthetic Genetics (San Diego, CA). In the case of large coding sequences, the oligonucleotide coding sequence can be synthesized through a series of cloning steps involving a tandem array of multiple oligonucleotide fragments corresponding to the coding sequence (Crea; Yoshio *et al.;* Eaton *et al.*). Oligonucleotide coding sequences can be amplified and isolated by standard recombinant procedures (Maniatis *et al.;* Ausubel *et al.*) or by polymerase chain reaction (Mullis; Mullis, *et al.*).

When the sequence of the membrane-associated antigen is known or partially known a specific antigen coding sequence may be isolated. Typically, the antigen coding sequence is isolated from a cDNA library, generated by the insertion of DNA fragments from a selected source into a vector. The cDMA library containing DNA fragments from a membrane antigen-containing source can be constructed using random fragments cDNA molecules generated from target RNA molecules. Such a cDNA library is generally constructed using a bacterial system (such as lambda gt10 (Promega, Madison WI)), but can also be constructed in a yeast or eukaryotic expression system using conventional techniques (Ausubel).

The library is screened (usually by hybridization; Ausubel, *et al.*; Maniatis, *et al.*) for the presence of the membrane-associated antigen DNA sequence, typically using as a probe an oligonucleotide having a known or consensus sequence hybridizable with the antigen coding region. The probe can carrying a number of detection moieties including radioisotopes, biotin and digoxigenin. Alternatively, when a nucleic acid probe sequence is not available, screening for clones carrying the coding region of interest can be carried out using, for example, immunoscreening (using "PROTOCLONE" lambda gt11 system, Promega; Young, *et al.;* Huynh, *et al.*).

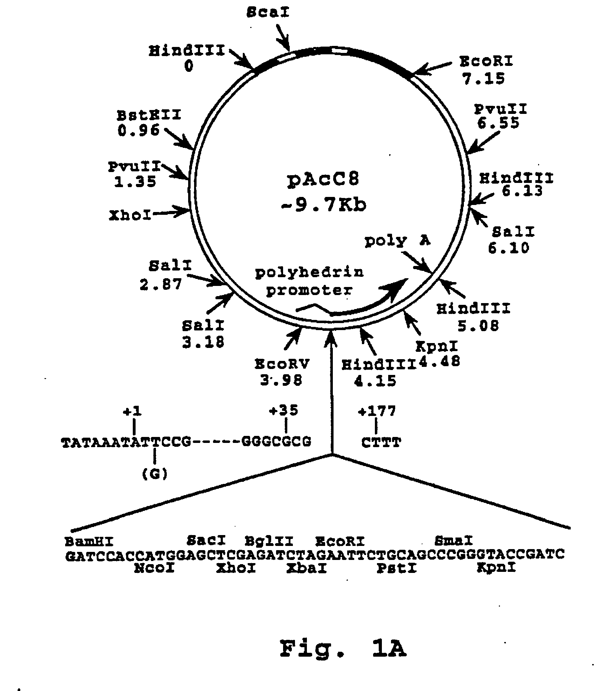

Coding regions are isolated from recombinant isolates giving positive signals (either by hybridization or immunological screening). Typically, DNA fragments containing the coding regions are isolated by restriction digestion followed by size fractionation and fragment purification. Such nucleic acid coding regions may then be processed for insertion into a baculoviral transfer vector, such as the vector pAcC8 (Figure 1A), as described in part B, below. Alternative baculovirus vectors are available including the vectors pVL1393 (Luckow *et al.*) and pAC3T3 (Summers et *al.*)*.*

Alternately, coding sequences can also be isolated using polymerase chain reaction (PCR) amplification (Mullis; Mullis, *et al*.). Primers useful for the PCR can be derived from any known nucleic acid sequence. If the exact sequence is not known degenerative primers can be used (Mullis; Mullis, *et al.*). Typically these primers are two nucleic acid sequences consisting of 8 or more colinear nucleotides, where the two sequences are separate by some defined distance, in order to generate a target sequence (Example 1), and are complementary to opposite strands.

A typical PCR cycle involved the following steps: melting at elevated temperature, followed by annealing, and extension. The reactions are repeated for 25-30 cycles. The PCR products can be digested with restriction enzymes and electrophoretically resolved using a preparative 1.5% agarose gel. clone-specific, amplified fragments are typically identified by electrophoretic gel size fractionation. The clone-specific DNA fragments are then recovered from the gel, for example, using the "GENE CLEAN" system (BIO 101, La Jolla CA). If necessary the DNA can be extracted with phenol and/or phenol:chloroform (1:1). Isolated DNA is ethanol precipitated. Following precipitation, the DNA is used for insertion into baculovirus expression vectors.

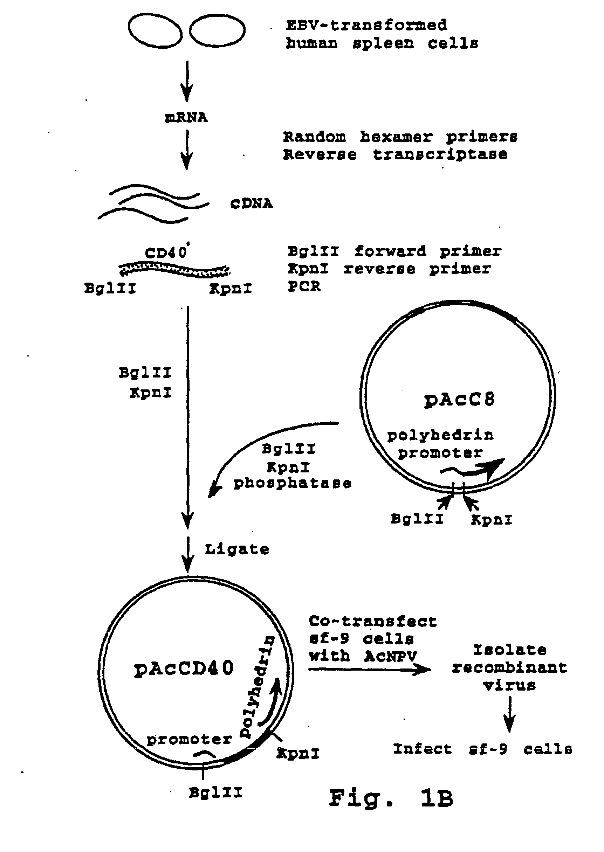

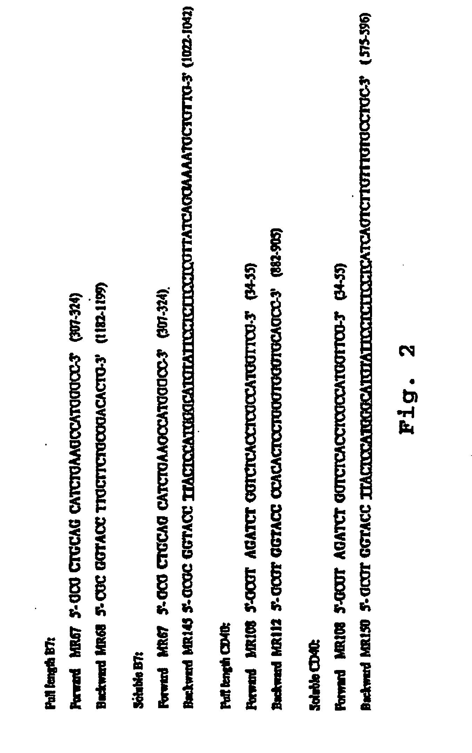

The generation of Sf9 cells expressing human CD40 or B7 antigens is schematically shown in Figure 1B. As shown, RNA is isolated from a population of Epstein-Barr virus (EBV)-transformed human spleen cells, using standard procedures (Chirgwin, *et al.*)*.* Total RNA is converted to cDNA using random hexamer priming, according to established methods, and as detailed in Example 1. The DNA molecule encoding the membrane-associated antigen molecules of interest is generated by PCR amplification, using forward and reverse primers having restriction sites for cloning at their 5' termini. Such cDNA primers, used in the preparation of coding regions for human CD40 and human B7 antigens are depicted in Figure 2. These primers were constructed on the basis of the published complete DNA coding sequences for antigens B7 and CD40 (Freeman *et al.,* 1989; Stamenkovic *et* al., 1989).

With continuing reference to Figure 1B, the cDNA is mixed with a forward primer and a reverse primer, in the presence of a thermostable polymerase, such as polymerase obtained from *Thermus aquaticus,* a mixture of equimolar deoxynucleotides, and a buffer system (Example 1). The mixture is subjected to amplification in a thermocycler, and PCR products obtained are subcloned in the polylinker of a baculovirus transfer vector. one such vector, pAcC8, is diagrammatically represented in Figure 1A. Any of a number of such baculoviral transfer vectors containing unique restriction endonuclease sites downstream of the polyhedrin promoter (Miller) can be utilized in the practice of the present invention: for *Autographica californica* nuclear polyhedrosis virus (AcNPV) (Wu, *et al*.; Matsuura, *et al*.; Takehara, *et al*.) or *Bombyx mori* nuclear polyhedrosis virus (pBmNPV) polyhedrin mRNA (Nyunoya, *et al*.; Sekine, *et al*.).

Before expression in baculovirus, DNA inserts are typically checked for PCR-induced mutations by sequencing analysis.

### B. Inserting an Antigen Coding Sequence into a Baculoviral Vector.

Insertion of the membrane-associated antigen coding region into a baculovirus vector is performed according to established procedures (Ausubel, et al.; Maniatis, *et al.;* Sambrook, *et al.*). Full length cDNAs encoding human B7 and human CD40 were generated by PCR using primers with restriction sites for cloning. The template for PCR amplification was cDNA generated from EBV-transformed human spleen B cell RNA. Briefly, an isolated DNA coding region is ligated into the baculoviral transfer vector or plasmid, such as a pAcC8 plasmid, so that the membrane-associated coding region is down-stream of the polyhedron promoter. The polyhedron gene ATG has been mutated to ATT (Figure 1A) to prevent translational initiation in recombinant clones that do not contain a coding sequence with a functional ATG. The resulting plasmid DNA is co-transfected with wild type baculovirus (AcNPV) into insect cells from *Spodoptera frugiperda* (Sf9 cells) to create recombinant virus particles, via *in vivo* recombination between the wild type virus and the recombinant vector, carrying the membrane-associated antigen gene.

Examples 1-2 describe the isolation of recombinant baculovirus vectors containing heterologous segments of DNA: pAcCD40 (encoding a full-length CD40 molecule), pAcCD40-ED/Glu (encoding the extracellular domain of CD40), pAcB7 (encoding a full-length B7 molecule) and pCcB7-ED/Glu (encoding the cellular domain of the B7 molecule).

### C. Infecting Insect Cells with Baculoviral Vectors.

The recombinant viruses described above were then used to co-infect insect cells (Example 2). These cells then expressed the antigens encoded by the heterologous DNA inserts.

Sf9 cells (*Spodoptera frugiperda*; Summers, *et al.*), at a density of 10⁶ cells/ml, were infected with recombinant virus. Recombinant baculovirus-infected Sf9 cells were identified and clonally purified (Summers *et al*.).

Cells expressing cell surface antigen were harvested after 48 hours and used for the immunization of host animals. For production of secreted recombinant proteins, the cells were harvested after 72 hours of culture.

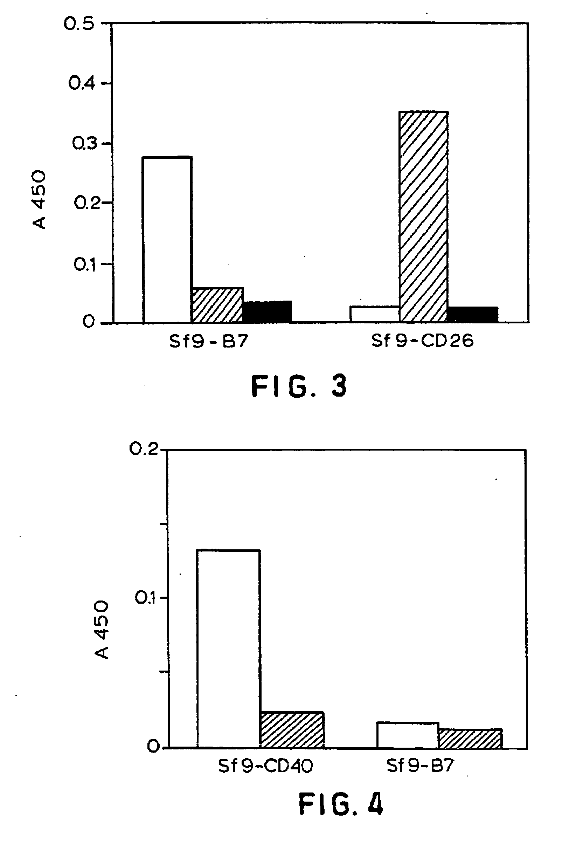

The expression of the recombinant molecules on the cell surface of the Sf9 cells (Example 3) was tested using an ELISA system (Harlow, et *al.*). Figure 3 shows that the anti-(B7) monoclonal antibody BB-1 reacted only with Sf9 cells infected with AcB7 virus, but not with Sf9 cells expressing human CD26. In contrast, the anti-(CD26) monoclonal antibody Ta-1 reacted only with the Sf9 cells expressing CD40. Figure 4 shows that the anti-(CD40) monoclonal antibody S2C6 reacted only with Sf9 cells expressing CD40, but not with Sf9 cells expressing B7. These results show the specificity of the method of the present invention for the production of selected membrane-associated antigens in the baculovirus system. These results also indicate that the membrane-associated antigens are exposed on the surface of the Sf9 cells.

Further, these results suggest that Sf9 cells expressing selected membrane-associated antigens can be used to screen sera and hybridoma supernatants for the presence of antibodies reactive against the selected antigen.

### D. Injecting Insect Cells Expressing the Membrane-Associated Antigen into a Host Animal.

Appropriate host animals for the production of polyclonal antibodies include, for example, rabbits, goats, sheep, guinea pigs, chimpanzees and dogs. One advantage of the present invention is that immunization adjuvants are generally not required.

Appropriate host animals for use in the production of monoclonal antibodies commonly include rats, hamsters and mice. However, in cases where it is desirable to produce antibodies that are immunologically closer to humans, sources of such antibodies may include higher primates such as chimpanzees. Fusion with a heteromyeloma fusion partner can be used for the generation of monoclonal antibodies (Carroll; Perkins, 1991). Such fusions can be achieved by a number of methods known in the art (Harlow, *et al*.) including exposure of mixed cells to polyethylene glycol and exposure of cells to strong electric field (electrofusion). Hybridomas are selected by growth in selective medium, then are tested for antigen specificity as described below.

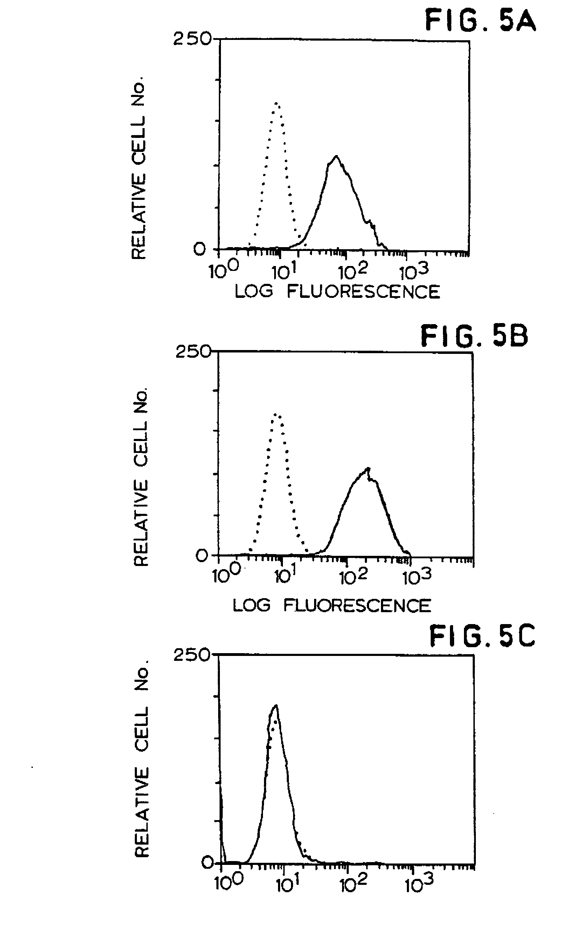

For the generation of monoclonal antibodies to CD40 and B7, mice were immunized (Example 5) with the Sf9 cells expressing these molecules on the cell surface. One week after the second immunization, the mice were bled and the sera were analyzed for the presence of specific antibodies using fluorescent cell staining of EBV-transformed B cells (Example 3). Figure 5 shows the results of the cell staining which indicate that mice immunized with Sf9 cells expressing CD40 or B7 had a serum titre against EBV-transformed B cell line ARC (American Type Culture Collection (A.T.C.C.), 12301 Parklawn Dr., Rockville MD 20852), which is positive for both CD40 and B7. In contrast, mice which were immunized with control Sf9 cells showed no reactivity with the ARC cells. The results indicate that host animals can be immunized with Sf9 cells expressing a membrane-associated antigen of choice and the immunization results in an immune response including antibodies against the recombinant antigen. The immunization does not result in antibodies cross-reactive with human proteins other than the recombinant human protein cloned in the Sf9 insect cells.

One mouse was given a final booster injection with CD40 expressing Sf9 cells and one with B7 expressing Sf9 cells. Three days after the booster injection, the spleens were removed and the splenocytes were fused with SP2/0 murine myeloma cells.

### E. Isolating and Immortalizing Specific Antibody-Producing Lymphocytes.

Antibody-producing lymphocytes for monoclonal antibody production are preferably B-lymphocytes, such as may be isolated from the bone marrow, spleen or lymph nodes of an immune host animal (Harlow, *et al.*)*.*

Alternatively, B-lymphocytes can be isolated from the peripheral circulation. In this case, blood samples are centrifuged, and are subjected to gradient separation techniques to produce a crude peripheral blood lymphocyte (PBL) mixture. Monocytes and T-lymphocytes are selectively depleted from this cell mixture according to established procedures (Mishell). Such remaining cells may be subjected to a selection procedure, such as a "panning" procedure, in which those cells having affinity for the antigen are concentrated by selective capture by an affinity matrix containing the antigen. In the context of the present invention, such a matrix might comprise a cell which expresses the membrane-associated antigen.

When B-lymphocytes are isolated from the circulation as described above, transformation with a transforming virus, such as Epstein-Barr virus, may be advantageous. Transformed cells (lymphoblastoids) are dispensed in subculture wells and maintained in culture for several weeks, prior to testing for specific antibody production. Cultures exhibiting such specific antibody production are expanded and fused with species-appropriate myeloma partner cells using one or more standard fusion protocols, including polyethylene glycol, as described above, or electrofusion. Methods for isolation and immortalization of B-lymphocytes from various sources are known in the art.

In experiments carried out in support of the present invention, splenocytes from immunized mice were fused with SP2/0 murine myeloma cells polyethylene glycol as previously described by de Boer *et al*. (1988). The hybridoma clones were processed as described in Example 6.

Table 1 (Example 6) gives a summary of the fusion data. After the CD40 fusion, only half of the cells were seeded in 480 wells. This resulted in 351 wells with hybridoma growth. After the B7 fusion, the cells were distributed in 960 wells and this fusion yielded 312 wells with hybridoma growth. Fourteen days after the fusions, supernatants of 12 wells were pooled and the pools were tested for the presence of antibodies reactive with ARC cells. FACS analysis revealed that 4 pools from the CD40 fusion and 1 pool from the B7 fusion were reactive with ARC cells. When individual supernatants from the positive pools were retested, 4 wells reactive with CD40 and 1 well reactive with B7 were identified. The cells from these positive wells were cloned by limiting dilution, and, after 3 rounds of cell growth, 4 stable anti-(CD40) hybridoma clones (CD40-3A8, CD40-3C6, CD40-5D12 and CD40-5 and 1 stable anti-(B7) hybridoma clone (B7-24) were established. These results indicate the ability to achieve stable hybridoma clones secreting monoclonal antibodies directed against a chosen membrane-associated antigen.

A number of methods for screening hybridoma fusions are available (Harlow, *et al.*), including: antibody capture, (i) using labeled antigen, e.g., radioactively labelled partially purified or purified antigen, (ii) whole or permeabilized cells, e.g., Sf9 cells expressing the recombinant antigen; and antigen capture, (i) antibody/antigen in solution, (ii) antibody/antigen solid phase.

### F. Testing the Specificity of the Monoclonal Antibodies.

EBV-transformed cells were used for the screening of the primary hybridoma supernatants and for screening of the subsequent products of the limiting dilution cloning. Several lines of evidence presented below suggest that 4 anti-(CD40) and 1 anti-(B7) monoclonal antibodies have been generated.

First, supernatants from all 5 hybridoma clones were reactive with ARC cells and other EBV-transformed B cell lines, but not with T cell lines HSB (A.T.C.C.) and CEMM (A.T.C.C.).

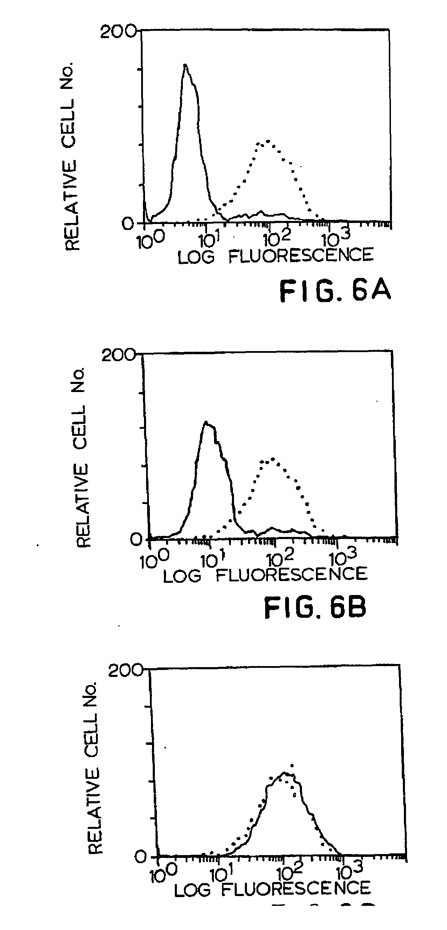

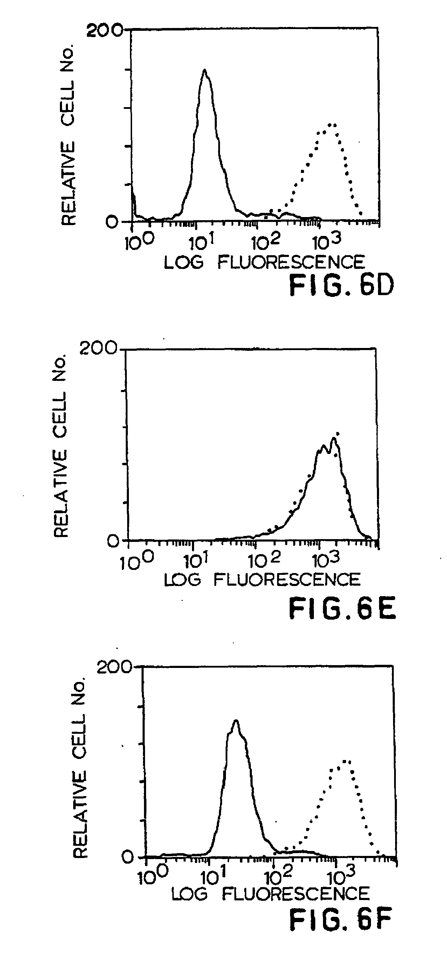

Second, competition binding experiments were performed using the monoclonal antibodies of the present invention and soluble forms of the target antigens (Example 7). Hybridoma supernatants were pre-incubated with soluble forms of CD40 and B7 (Example 7). Subsequently, the mixtures were added to ARC cells for fluorescent cell staining. The results of the competition experiment are shown in Figure 6. The data show that soluble B7, but not soluble CD40, could block the binding of anti-(B7) monoclonal antibody B7-24 to ARC cells. Conversely, soluble CD40, but not soluble B7, could block the binding of anti-(CD40) monoclonal antibody CD40-3A8 to ARC cells. Similar results were obtained with the other 3 anti-(CD40) monoclonal antibodies. Furthermore, the effects of soluble CD40 on the anti-(CD40) monoclonal antibodies and the effect of soluble B7 on the anti-(B7) monoclonal antibody was concentration dependent. Decreasing the amount of soluble protein resulted in decreased blocking of binding of the antibodies to ARC cells.

For further analysis, the anti-(CD40) and anti-(B7) monoclonal antibodies were tested for their ability to bind to tonsillar B cells (Example 8). Table 2 shows that 89-95 percent of freshly isolated tonsillar B cells stained positive with the four anti-(CD40) monoclonal antibodies. About the same percentage of cells was positive with anti-(CD40) monoclonal antibody G28.5 (Clark, *et al.*). Table 3 shows that 12-17 percent of freshly isolated tonsillar B cells stained positive with anti-(B7) monoclonal antibody B7-24. However, when tonsillar B cells were cultured for 5 days in the presence of immobilized anti-(IgM) antibodies and IL-2, the percentage of cells positive for B7-24 increased up to about 25 percent.

Furthermore, when tonsillar B cells were stimulated with anti-(IgM) antibodies and IL-2, not only did the number of B cells positive for B7-24 increase, but there was also a significant increase in the amount of fluorescent staining per cell, indicating that the expression of B7 was increased after stimulation.

The above data indicate that the method of the present invention provides a way to isolate monoclonal antibodies which are specifically reactive with membrane-associated antigens. The monoclonal antibodies obtained by the method of the present invention can be typed as previously described (Harlow, *et al.*).

### G. Utility

For the production of monoclonal antibodies it is optimal to immunize mice with purified material. However, purification of membrane antigens requires specialized and complex techniques, and furthermore, extraction from the membrane may alter the structure of the molecule. In addition, solubilization of proteins often decreases their immunogenicity. Therefore, most monoclonal antibodies to cell surface antigens have been obtained after immunization with mice with whole cells or membrane fractions. In many cases, specific lymphocyte subsets have been injected into mice resulting in panels of monoclonal antibodies. These antibodies have been used to isolate and characterize the antigen that they bound. When mice are immunized with whole cells, antibodies to a large number of different molecules are generated. It is therefore difficult to use the same cells for the screening of specific antibody production by the hybridoma clones.

To circumvent the above-mentioned problem, the method of the present invention involves the expression of membrane-associated antigens in insect cells and the use of these insect cells to immunize host animals. Since the introduction of PCR technology (Saiki *et al.,* 1985; saiki *et al.,* 1988; Mullis; Mullis, *et al*.), it has become relatively straight-forward to clone cDNAs for proteins whose coding nucleic acid coding sequence has been published. One can use PCR primers spanning the complete coding region only, and incorporate restriction sites in these primers to facilitate cloning into expression vectors.

It has been shown that human intracellular, secreted and transmembrane proteins can be expressed at high levels in insect cells when expressed in the cells under the regulation of the non-essential baculovirus gene for the polyhedrin protein (Webb *et al.,* 1989; reviewed by Luckow, 1990). Experiments performed in support of the present invention have shown that only 2 injections with 5×10⁶ Sf9 cells expressing human CD40 or human B7 gave good serum titres against these antigens. Furthermore, the insect cells themselves did not evoke an immune response cross reactive with human cells. This enabled the use EBV transformed B cells for the screening of specific antibody production by the hybridoma clones, with minimal risk of obtaining false positives. All of the positive primary wells obtained by the method of the present invention were in fact specific for the antigen that was used for immunization.

Antibodies obtained by the method of the present invention, directed against membrane-associated antigens, are advantageous for use as diagnostic agents for the detection of the membrane-associated antigen. For example, antibodies directed against cell-surface marker proteins or viral proteins protruding from the cell surface.

One diagnostic configuration involves use of anti-viral antibodies capable of detecting viral specific antigens. The antigens may be detected, for example, using an antigen capture assay where viral antigens present in candidate serum samples are reacted with an antigen-specific monoclonal or polyclonal antibody. The antibody is bound to a solid substrate and the antigen is then detected by a second, different labelled antibody directed against the anti-viral antibody.

The anti-viral antibodies obtained by the method of the invention can be used as a means of enhancing an anti-viral immune response since antibody-virus complexes are typically recognized by macrophages and other effector cells. The antibodies can be administered in amounts similar to those used for other therapeutic administrations of antibody. For example, pooled gamma globulin is administered at 0.02-0.1 ml/lb body weight during the early incubation of viral diseases such as rabies, measles and hepatitis B to interfere with viral entry into cells. Thus, antibodies reactive with a membrane-associated viral antigen can be passively administered alone, in a "cocktail" with other anti-viral antibodies, or in conjunction with another anti-viral agent to enhance the immune response and/or the effectiveness of an antiviral drug.

### III. Compositions Using Antibodies

This invention contemplates the use of antibodies such as those made by the above-described method. The antibodies of the current invention either (1) bind to a human CD40 antigen on the surface of a human B cell and do not stimulate the growth of differentiation of the B cell or (2) bind to the B7 antigen. These antibodies may, however, be polyclonal antibodies, monoclonal antibodies, humanized antibodies, single-chain antibodies, and fragments thereof. The anti-CD40 antibodies are used alone, while the anti-B7 antibodies may be used in a composition that also contains an immunosuppressive agent.

### A. Antibody Preparation

Monoclonal antibodies 5D12, 3A8, 3C6, and B7-24 are prepared as in section II of the detailed description and Examples 1-7 herein. Other monoclonal antibodies of the invention may be prepared similarly, or as follows. First, polyclonal antibodies are raised against the CD40 or B7antigen. Second, monoclonal antibodies specific for CD40 or B7 are selected.

### 1. Polyclonal Sera

Polyclonal sera may be prepared by conventional methods. In general, a solution containing the CD40 or B7 antigen is first used to immunize a suitable animal, preferably a mouse, rat, rabbit or goat. Rabbits and goats are preferred for the preparation of polyclonal sera due to the volume of serum obtainable, and the availability of labeled anti-rabbit and anti-goat antibodies. Immunization is generally performed by mixing or emulsifying the antigen-containing solution in saline, preferably in an adjuvant such as Freund's complete adjuvant, and injecting the mixture or emulsion parenterally (generally subcutaneously or intramuscularly). A dose of 50-200 µg/injection is typically sufficient. Immunization is generally boosted 2-6 weeks later with one or more injections of the protein in saline, preferably using Freund's incomplete adjuvant. One may alternatively generate antibodies by in vitro immunization using methods known in the art, which for the purposes of this invention is considered equivalent to *in vivo* immunization.

Polyclonal antisera are obtained by bleeding the immunized animal into a glass or plastic container, incubating the blood at 25ºC for one hour, followed by incubating at 4ºC for 2-18 hours. The serum is recovered by centrifugation (e.g., 1,000 x g for 10 minutes). About 20-50 ml per bleed may be obtained from rabbits.

### 2. Monoclonal Antibodies

Monoclonal antibodies are prepared using the method of Kohler and Milstein, Nature (1975) 256:495-96, or a modification thereof. Typically, a mouse or rat is immunized as described above. However, rather than bleeding the animal to extract serum, the spleen (and optionally several large lymph nodes) are removed and dissociated into single cells. If desired, the spleen cells may be screened (after removal of nonspecifically adherent cells) by applying a cell suspension to a plate or well coated with the protein antigen. B-cells expressing membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (e.g., hypoxanthine, aminopterin, thymidine medium, "HAT"). The resulting hybridomas are plated by limiting dilution, and are assayed for the production of antibodies which bind specifically to the desired immunizing cell-surface antigen (and which do not bind to unrelated antigens). The selected mAb-secreting hybridomas are then cultured either in vitro (e.g., in tissue culture bottles or hollow fiber reactors), or *in vivo* (as ascites in mice).

If desired, the antibodies (whether polyclonal or monoclonal) may be labeled using conventional techniques. Suitable labels include fluorophores, chromophores, radioactive atoms (particularly ³²P and ¹²⁵I), electron-dense reagents, enzymes, and ligands having specific binding partners. Enzymes are typically detected by their activity. For example, horseradish peroxidase is usually detected by its ability to convert 3,3',5,5'-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefor. Other specific binding partners include biotin and avidin or streptavidin, IgG and protein A, and the numerous receptor-ligand couples known in the art. It should be understood that the above description is not meant to categorize the various labels into distinct classes, as the same label may serve in several different modes. For example, ¹²³I may serve as a radioactive label or as an electron-dense reagent. HRP may serve as enzyme or as antigen for a mAb. Further, one may combine various labels for desired effect. For example, mAbs and avidin also require labels in the practice of this invention: thus, one might label a mAb with biotin, and detect its presence with avidin labeled with ¹²⁵I, or with an anti-biotin mAb labeled with HRP. Other permutations and possibilities will be readily apparent to those of ordinary skill in the art, and are considered as equivalents within the scope of the instant invention.

### IV. Compositions Including Immunosuppressive Agents

A composition of the current invention comprises two components which together are therapeutically effective in preventing or treating graft rejection, GVHD, or rheumatoid arthritis. The two components are: (1) a molecule that binds to the B7 antigen such as MAb B7-24; and (2) an immunosuppressive agent. Molecules that bind to the B7 antigen include CD28, CTLA4, CTLA4Ig and anti-B7 antibodies as described in Section III above.

The anti B7antibodies of the invention (or other molecules that bind to the B7 antigen) are given in combination with one or more immunosuppressive agents. Immunosuppressive agents are agents that block or inhibit the activation or proliferation of T cells. The immunosuppressive agents according to this invention include cyclosporin A (CsA), corticosteroids (methotrexate, prednisolone, dexamethasone), FK506, and rapamycin. Preferably the immunosuppressive agent is cyclosporin A, FK506 or a corticosteroid, most preferably cyclosporin A

### V. CD40 Antigen Epitopes

The CD40 antigen epitopes of this invention are molecules that are immunoreactive with anti-CD40 monoclonal antibodies whose binding to a human CD40 antigen located on the surface of a human B cell prevents the growth or differentiation of the B cell. That is, such epitopes compete with the binding of said antibodies to the CD40 antigen. Systematic techniques for identifying these epiotpes are known in the art, as described by H.M. Geysen in U.S. Patent No. 4,708,871, which is incorporated herein by reference. Typically these epitopes are short amino acid sequences. These sequences may be embedded in the sequence of longer peptides or proteins, as long as they are accessible.

The epitopes of the invention may be prepared by standard peptide synthesis techniques, such as solid-phase synthesis. Alternatively, the sequences of the invention may be incorporated into larger peptides or proteins by recombinant methods. This is most easily accomplished by preparing a DNA cassette which encodes the sequence of interest, and ligating the cassette into DNA encoding the protein to be modified at the appropriate site. The sequence DNA may be synthesized by standard synthetic techniques, or may be excised from the phage pIII gene using the appropriate restriction enzymes.

Epitopes identified herein may be prepared by simple solid-phase techniques. The minimum binding sequence may be determined systematically for each epitope by standard methods, for example, employing the method described by H.M. Geysen, U.S. Pat. No. 4,708,871. Briefly, one may synthesize a set of overlapping oligopeptides derived from the CD40 antigen bound to a solid phase array of pins, with a unique oligopeptide on each pin. The pins are arranged to match the format of a 96-well microtiter plate, permitting one to assay all pins simultaneously, e.g., for binding to an anti-CD40 monoclonal antibody. Using this method, one may readily determine the binding affinity for every possible subset of consecutive amino acids.

Analogs of the invention are also prepared by standard solid-phase methods, and those methods described in PCT application US91/04282.

### VI. Formulations and Methods of Administration

The antibodies and compositions of this invention are administered at a concentration that is therapeutically effective to halt transplant rejection, or prevent or treat (1) GVHD or rheumatoid arthritis, or (2) antibody-mediated diseases such as allergies, SLE, PBC and ITP. To accomplish this goal, the antibodies or compositions may be formulated using a variety of acceptable excipients known in the art. Typically, the antibodies or compositions are administered by injection, either intravenously or intraperitoneally. Methods to accomplish this administration are known to those of ordinary skill in the art. It may also be possible to obtain compositions which may be topically or orally administered, or which may be capable of transmission across mucous membranes.

Before administration to patients, formulants may be added to the antibodies. A liquid formulation is preferred. For example, these formulants may include oils, polymers, vitamins, carbohydrates, amino acids, salts, buffers, albumin, surfactants, or bulking agents. Preferably carbohydrates include sugar or sugar alcohols such as mono, di, or polysaccharides, or water soluble glucans. The saccharides or glucans can include fructose, dextrose, lactose, glucose, mannose, sorbose, xylose, maltose, sucrose, dextran, pullulan, dextrin, alpha and beta cyclodextrin, soluble starch, hydroxethyl starch and carboxymethylcellulose, or mixtures thereof. Sucrose is most preferred. "Sugar alcohol" is defined as a C₄ to C₈ hydrocarbon having an -OH group and includes galactitol, inositol, mannitol, xylitol, sorbitol, glycerol, and arabitol. Mannitol is most preferred. These sugars or sugar alcohols mentioned above may be used individually or in combination. There is no fixed limit to amount used as long as the sugar or sugar alcohol is soluble in the aqueous preparation. Preferably, the sugar or sugar alcohol concentration is between 1.0 w/v% and 7.0 w/v%; more preferable between 2.0 and 6.0 w/v%. Preferably amino acids include levorotary (L) forms of carnitine, arginine, and betaine; however, other amino acids may be added. Preferred polymers include polyvinylpyrrolidone (PVP) with an average molecular weight between 2,000 and 3,000, or polyethylene glycol (PEG) with an average molecular weight between 3,000 and 5,000. It is also preferred to use a buffer in the composition to minimize pH changes in the solution before lyophilization or after reconstitution. Most any physiological buffer may be used, but citrate, phosphate, succinate, and glutamate buffers or mixtures thereof are preferred. Most preferred is a citrate buffer. Preferably, the concentration is from 0.01 to 0.3 molar. Surfactants that can be added to the formulation are shown in EP Nos. 270,799 and 268,110.

Additionally, antibodies can be chemically modified by covalent conjugation to a polymer to increase their circulating half-life, for example. Preferred polymers, and methods to attach them to peptides, are shown in U.S. Patent Nos. 4,766,106; 4,179,337; 4,495,285; and 4,609,546 which are all hereby incorporated by reference in their entireties. Preferred polymers are polyoxyethylated polyols and polyethylene glycol (PEG). PEG is soluble in water at room temperature and has the general formula: R(O-CH₂-CH₂)ₙO-R where R can be hydrogen, or a protective group such as an alkyl or alkanol group. Preferably, the protective group has between 1 and 8 carbons, more preferably it is methyl. The symbol n is a positive integer, preferably between 1 and 1,000, more preferably between 2 and 500. The PEG has a preferred average molecular weight between 1000 and 40,000, more preferably between 2000 and 20,000, most preferably between 3,000 and 12,000. Preferably, PEG has at least one hydroxy group, more preferably it is a terminal hydroxy group. It is this hydroxy group which is preferably activated to react with a free amino group on the inhibitor. However, it will be understood that the type and amount of the reactive groups may be varied to achieve a covalently conjugated PEG/antibody of the present invention.

Water soluble polyoxyethylated polyols are also useful in the present invention. They include polyoxyethylated sorbitol, polyoxyethylated glucose, polyoxyethylated glycerol (POG), etc. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG. The structure for POG is shown in Knauf et al., 1988, J. Bio. Chem. 263:15064-15070, and a discussion of POG/IL-2 conjugates is found in U.S. Patent No. 4,766,106, both of which are hereby incorporated by reference in their entireties.

Another drug delivery system for increasing circulatory half-life is the liposome. Methods of preparing liposome delivery systems are discussed in Gabizon et al., Cancer Research (1982) 42:4734; Cafiso, Biochem Biophys Acta (1981) 649:129; and Szoka, Ann Rev Biophys Eng (1980) 9:467. Other drug delivery systems are known in the art and are described in , e.g., Poznansky et al., DRUG DELIVERY SYSTEMS (R.L. Juliano, ed., Oxford, N.Y. 1980), pp. 253-315; M.L. Poznansky, Pharm Revs (1984) 36:277.

After the liquid pharmaceutical composition is prepared, it is preferably lyophilized to prevent degradation and to preserve sterility. Methods for lyophilizing liquid compositions are known to those of ordinary skill in the art. Just prior to use, the composition may be reconstituted with a sterile diluent (Ringer's solution, distilled water, or sterile saline, for example) which may include additional ingredients. Upon reconstitution, the composition is preferably administered to subjects using those methods that are known to those skilled in the art.

As stated above, the anti-CD40 antibodies and compositions of this invention are used to treat human patients to prevent or treat antibody-mediated diseases such as allergies, SLE, PBC and ITP. The anti-B7 antibodies are preferably used to prevent or treat transplant rejection, GVHD or rheumatoid arthritis. The preferred route of administration for these antibodies is parenteral. In parenteral administration, the compositions of this invention will be formulated in a unit dosage injectable form such as a solution, suspension or emulsion, in association with a pharmaceutically acceptable parenteral vehicle. Such vehicles are inherently nontoxic and nontherapeutic. Examples of such vehicles are saline, Ringer's solution, dextrose solution, and Hanks' solution. Nonaqueous vehicles such as fixed oils and ethyl oleate may also be used. A preferred vehicle is 5% dextrose in saline. The vehicle may contain minor amounts of additives such as substances that enhance isotonicity and chemical stability, including buffers and preservatives.

The dosage and mode of administration will depend on the individual. Generally, the compositions are administered so that antibodies are given at a dose between 1 µg/kg and 20 mg/kg, more preferably between 20 µg/kg and 10 mg/kg, most preferably between 1 and 7 mg/kg. Preferably, it is given as a bolus dose, to increase circulating levels by 10-20 fold and for 4-6 hours after the bolus dose. Continuous infusion may also be used after the bolus dose. If so, the antibodies may be infused at a dose between 5 and 20 µg/kg/minute, more preferably between 7 and 15 µg/kg/minute.

The following examples illustrate, but in no way are intended to limit, the present invention.

### Materials and Methods

Iscove's modification of Dulbecco's Eagle medium (IMDM) and foetal bovine serum were obtained from JR Biosciences (Lenexa, KS); penicillin and streptomycin were obtained from Irvine (Santa Ana, CA); and polyethylene glycol (mol. wt. 1500) was obtained from Boehringer Mannheim (Indianapolis, IN).

Culture Media. SP2/0 murine myeloma cells, hybridoma cells, purified T cells, EBV-transformed B cells and cell lines were cultured in IMDM supplemented with streptomycin (200 µg/ml), penicillin (200 U/ml) and 10% heat inactivated foetal bovine serum (complete IMDM). The Sf9 insect cells were cultured in shaker flasks agitated (125-150 rpm) in medium described by Maiorella *et al.* (1989) supplemented with 0.5% foetal bovine serum. 3T6-FcγRII cells were cultured in medium consisting of 50% Dulbecco's modified Eagle's medium and 50% HAM-F10 medium, supplemented with aminopterin (0.2 µg./ml), thymidine (5 µg/ml), xanthine (10 µg/ml), hypoxanthine (15 µg/ml), mycophenolic acid (20 µg/ml) deoxycytidine (2.3 µg/ml), and 10% heat-inactivated fetal bovine serum (complete DME/HAM-F10). 3T6-FcγRII/B7 cells were cultured in complete DME/HAM-F10 medium containing 400 µg/ml G418 (Gibco).

Cells and Cell Lines. Peripheral blood mononuclear cells were isolated from heparinized blood (obtained from healthy volunteers) by Ficoll-Hypaque density centrifugation. T cells were enriched by depleting monocytes and B cells using Lymphokwik (Lambda, California) (1 λ). The EBV-transformed B cell line ARC and the P815 cell line, a NK-resistant murine mastocytoma cell line that expresses FcγRII and FcγRIII (Ra, C. et al., Nature (1989) 341:752), were obtained from the ATCC (Rockville, MD). 3T6-FcγRII, the mouse fibroblast cell line expressing CD32, the human FcγRII high responder allele, as described by Warmerdam, P.A.M. et al., J. Exp. Med. (1990) 172:19, was kindly provided by Dr. J. van de Winkel (University Hospital, Utrecht, The Netherlands). The mutant mouse thymoma EL-4 subclone EL4B5 was a gift of Dr. R.H. Zubler, Hôpital Cantonal Universitaire, Geneva. Mouse 3T6 transfectant cells expressing hybrid molecules of the HR (high responder) allelic form of human FcγRIIa were a gift of Dr. P.A.M. Warmerdam, Department of Experimental Immunology, University Hospital Utrecht, Utrecht, The Netherlands. Warmerdam et al., J. Immunol. (1991) 147:1338. Both cell lines were cultured in Iscove's Modified Dulbeeco's Medium (IMDM), supplemented with gentamycin (80 µg/ml) and 10% heat-inactivated fetal calf serum (FCS) (Hyclone, Logan, Utah). To avoid possible loss of B cell activating capacity, every 4 to 8 weeks a new batch of EL4B5 cells was thawed. The cell lines were periodically tested for mycoplasma contamination by the use of a ³H-labelled DNA probe for mycoplasma ribosomal RNA (GenProbe, San Diego, CA) and were free of mycoplasma during the course of the experiments.

Human B Lymphocytes. B lymphocytes were isolated from tonsils obtained from children undergoing tonsillectomies, essentially as described in De Groot et al., Lymphokine Research (1990) 9:321. Briefly, the tissue was dispersed with scalpel blades, phagocytic and NK cells were depleted by treatment with 5 mM L-leucine methyl ester and T cells were removed by one cycle of rosetting with sheep erythrocytes (SRBC) treated with 2-aminoethyl isothiouronium bromide. The purity of the resulting B lymphocyte preparations was checked by indirect immunofluorescent labelling with anti-(CD20) mAb B1 (Coulter Clone, Hialeah, FA) or anti-(CD3) mAb OKT3 (Ortho, Raritan, NJ) and a FITC-conjugated F(ab')₂ fragment of rabbit anti-(mouse Ig) (Zymed, San Francisco, CA), and FACS analysis. The B cell preparations contained (mean ± SD of 6 isolations): 95 ± 4% CD20-positive cells and 2 ± 1% CD3-positive cells.

Antibodies. Anti-(human B7) monoclonal antibody BB-1 (Yokochi *et al.,* 1982) was obtained from Dr. E.A. Clark (University of Washington, Seattle, WA) and was used as purified antibody. Anti-(human CD40) monoclonal antibody G27.5 (Clark *et al.,* 1986) was obtained from Dr. J.A. Ledbetter (Oncogen Corporation, Seattle, WA) and was used as purified antibody. Anti-(CD40) monoclonal antibody S2C6 (Paulie et al., 1985) was obtained from Dr. S. Paulie (University of Stockholm, Stockholm, Sweden) and was used as purified antibody. Anti-(human CD26) monoclonal antibody Ta-1 and anti-(CD20) monoclonal antibody B1 were obtained from Coulter (Hialeah, FL). Anti-(CD3) monoclonal antibody OKT3 was obtained from Ortho (Raritan, NJ), and the anti-(LeuM3) monoclonal antibody was obtained from Becton-Dickinson (San Jose, CA). Anti-(IgM) antibodies coupled to beads (Immunobeads) were obtained from Bio-Rad (Richmond, CA).

Anti-B7 Mab B7-24 (IgG2a, x), and anti-CD40 mabs 5D12, 3C6 and 3A8 were obtained as described in Section II above, and used as purified antibodies. Anti-CD3 Mab CLB-T3/4.1 (IgG1, x) was used as diluted tissue culture supernatant and was kindly supplied by Dr. L. Aarden (Central Laboratory of the Red Cross Blood Transfusion Service, Amsterdam, The Netherlands). Anti-CD3 Mab UCHT1 (IgG, x) was used as purified antibody and as a gift of Dr. P. Beverley (Imperial Research Cancer Fund, London, UK). Anti-CD72 Mab WL225 (IgG2a, x) was used as purified antibody and was a gift of Dr. K. Thielemans (Vrije Universiteit Brussel, Belgium). The anti-ICAM-1 Mab 84H10 was used as diluted ascites fluid. Anti-CD40 mAb S2C6 was a gift of Dr. S. Paulie (University of Stockholm, Sweden). Paulie et al., J. Immunol. (1989) 142:590. Anti-CD40 mAb G28.5 was donated by Dr. J.A. Ledbetter (Oncogen Corporation, Seattle, WA, USA). Clark et al., PNAS (USA) (1986) 83:4494. Control antibodies were: anti-(β-glucocerebrosidase) mAb 8E4 (IgG1), Barneveld et al., Eur. J. Biochem. (1983) 134:585, and myeloma immunoglobulins MOPC-21 (IgG1) and MOPC-141 (IgG2b) (sigma, St. Louis, MO). All mAb were used as purified antibody preparations. hCD40.Hµ fusion protein was a gift of Dr. P. Lane (Basel Institute for Immunology, Basel, Switzerland) and was used as a 5x concentrated supernatant of transfected J558L cells. Lane et al., Eur. J. Immunol. (1992) 22:2573.

The monoclonal antibodies of the present invention can be labeled, by standard methods, using a number of reporter moieties, including the following: fluorescent labels (fluorescein (FITC), R-phycoerythrin, rhodamine (TMRITC), rhodamine 600 (XRITC), "TEXAS RED," and the like, commonly avidin linked); radioactive moieties (¹²⁵I and the like); light-emitting (luciferase and the like); enzymatic (horseradish peroxidase, alkaline phosphatase, glucose oxidase, β-galactosidase, and the like). Further, reporter antibodies (antibodies which have binding specificity for the monoclonal antibodies of the present invention, e.g., goat anti-mouse IgG) can also use the above-listed labelling moieties.

Enzymes and Oligonucleotides. *E. coli* DNA polymerase I (Klenow fragment) was obtained from Boehringer Mannheim Biochemicals (BMB) (Indianapolis, IN). T4 DNA ligase and T4 DNA polymerase were obtained from New England Biolabs (Beverly, MA); Nitrocellulose filters are obtained from Schleicher and Schuell (Keene, NH). Synthetic oligonucleotide linkers and primers were prepared using commercially available automated oligonucleotide synthesizers. Alternatively, custom designed synthetic oligonucleotides may be purchased, for example, from Synthetic Genetics (San Diego, CA). cDNA synthesis kit and random priming labeling kits are obtained from Boehringer-Mannheim Biochemical (BMB, Indianapolis, IN). Oligonucleotide sequences encoding peptides can be either synthesized as described above. Alternatively, peptides can be synthesized directly by standard in vitro techniques (Applied Biosystems, Foster City CA).

Common manipulations involved in polyclonal and monoclonal antibody work, including antibody purification, were performed by standard procedures (Harlow, et *al.*).

Fluorescent Cell Staining (FACS) Assay. Cells (10⁶/sample) were incubated in 10 µl primary antibody (10 µl/ml in PBS-BSA or HBSS (Hanks' Balanced Salt Solution, Gibco/BRL) supplemented with 1% BSA and 0.05% sodium azide) for 20 minutes at 4ºC. After 3 t washes with PBS-BSA or HBSS-BSA, the cells were incubated in 100 µl FITC-labeled F_{ab'2} fragments of goat anti-(mouse IgG) antibodies (Jackson, West Grove, PA) for 20 minutes at 4ºC. After 3 washes with PBS-BSA or HBSS-BSA and 1 wash with PBS, the cells were resuspended in 0.5 ml PBS. Analyses were performed with a FACSSCAN V (Becton Dickinson, San Jose, CA).

### B7-Mediated T Cell Proliferation Assay.

Purified T cells were cultured with 3T6 fibroblasts transfected with the FcγRIIa high responder allele and the B7 molecule (de Boer, M. et al., Eur. J. Immunol. (1992) 22:3071-3075). Proliferation was measured by ³H-thymidine incorporation. Briefly, 4 x 10⁴ T cells were cultured with 10⁴ irradiated (2500 rads) 3T6-FcγRII/B7 cells in 96-well flat-bottom tissue culture plates in 200 µl/well complete IMDM with or without anti-CD3 Mab CLB-T3/4.1. During the last 16 hours of a 72 hour culture period, the cells were pulsed with 1 µCi/well ³H-thymidine. Proliferation of T cells is expressed as the mean cpm of triplicate wells.

Cytotoxic T Cell Assay. Purified T cells were cultured with 3T6 fibroblasts transfected with the FcγRIIa high responder allele and the B7 molecule. Briefly, 10⁶ T cells were cultured with 0.2 x 10⁶ irradiated (2500 rads) 3T6-FcγRII/B7 cells in 24-well flat-bottom tissue culture plates in 1 ml/well complete IMDM in the presence of anti-CD3 Mab UCHT1 for 3-4 days. The cytotoxic activity of the lymphocytes was analyzed in an anti-CD3-redirected cytotoxicity assay as described below.

### Mixed Lymphocyte Culture (MLC) Assay.

Proliferation of purified T cells was measured in mixed lymphocyte cultures (MLC) using the EBV-transformed B cell line ARC as stimulator cells. 5 x 10⁴ T cells were cultured with 5 x 10⁴ irradiated (5000 rads) stimulator cells in 96-well round-bottom tissue culture plates (Corning) in 200 µl/well complete IMDM medium. During the last 16 hours of a 72 hour culture period, the cells were pulsed with 1 µCi/well ³H-thymidine. Proliferation of T cells is expressed as the mean cpm of triplicate wells. For secondary MLCs, cells were stimulated as described above for primary MLC. The T cell blasts for secondary MLC were generated in 5- to 7-day primary MLC, with subsequent culture in the absence of the stimulator cells for 2-4 days. The cytotoxic activity of T cells generated in primary or secondary MLC was analyzed in an anti-CD3-redirected cytotoxicity assay using the mouse P815 cells as described below. Alternatively, the EBV-transformed B cells used to induce the CTL activity served as target cells.

Cytotoxicity Assay. CTL activity was determined in a 4 hour target cell lysis assay using P815 murine mastocytoma cells or ARC EBV-transformed B cells as targets. In the case of the P815 target cells the CTLs were bridged non-specifically to the target cells using the anti-CD3 Mab OKT3 at 2 µg/ml. When the ARC cells were used as target cells, only the alloantigen-specific CTLs participate in the killing process. 10⁶ target cells were incubated with 200 µCi of ⁵¹Cr-sodium chromate (Amsersham International) for one hour and subsequently washed. The CTL assays were performed in 96-well V-bottom microtiter plates using 5000 ⁵¹Cr-labelled target cells with different amounts of effector cells in a total volume of 200 µl/well. Four wells were filled with 5 x 10³ target cells in 200 µl medium alone, and four wells with 5 x 10³ target cells in 100 µl medium and 100 µl saponin (for evaluation of spontaneous and maximal release, respectively). In the case of the P815 cells, three wells were filled with effector cells and target cells in the absence of anti-CD3 Mab (to determine the background experimental lysis). Three other wells also contained the anti-CD3 Mab at 2 µg/ml in order to determine the total lysis in the presence of anti-CD3. The plates were centrifuged for 10 minutes at 200 x g and incubated for four hours at 37ºC. Afterwards, 100 µl of the supernatant of each well was counted in a gamma counter. Results are expressed as percentage of anti-CD3-dependent specific release with the P815 target cells, or as a percentage of alloantigen-specific release with the ARC target cells.

B-Cell Proliferation Assay. B cells (4 x 10⁴ per well) were cultured in 200 µl IMDM supplemented with 10% fetal calf serum in flat bottom 96-well microtiter plates. B cells were stimulated by addition of immobilized anti-(IgM) antibodies (Immunobeads; 5 µg/ml; BioRad, Richmond, CA). Where indicated 100 U/ml recombinant IL-2 was added. Varying concentrations of mAbs were added at the onset of the microcultures and proliferation was assessed at day 3 by measurement of the incorporation of [³H]-thymidine after 18 hour pulsing.

### Banchereau-Like B-Cell Proliferation Assay.

For testing the ability of anti-CD40 mAbs to stimulate B-cell proliferation in a culture system analogous to that described by Banchereau et al., Science (1989) 251:70, mouse 3T6 transfectant cells expressing the HR allellic form of human FcγRII were used. B cells (2 x 10⁴ per well) were cultured in flat-bottom microwells in the presence of 1 x 10⁴ transfectant cells (irradiated with 5000 Rad) in 200 µl IMDM supplemented with 10% fetal calf serum and 100 U/ml recombinant IL-4. Before addition of the B cells, the 3T6 cells were allowed to adhere to the culture plastic for at least 5 hours. Anti-CD40 mAbs were added at concentrations varying from 15 ng/ml to 2000 ng/ml and proliferation of B cells was assessed by measurement of thymidine incorporation at day 7, upon 18 hour pulsing with [³H]-thymidine.

B-Cell Activation Assay with EL4B5 Cells. B cells (1000 per well) were cultured together with irradiated (5000 Rad) EL4B5 cells (5 x 10⁴ per well) in flat bottom microtiter plates in 200 µl IMDM supplemented with 10% heat-inactivated fetal calf serum, 5 ng/ml phorbol-12-myristate 13-acetate (Sigma) and 5% human T-cell supernatant. MAbs were added at varying concentrations at the onset of the cultures and thymidine incorporation was assessed at day 6 after 18 hour pulsing with [³H]-thymidine. For the preparation of T-cell supernatant, purified T cells were cultured at a density of 10⁶/ml for 36 hours in the presence of 1 µg/ml PHA and 10 ng/ml PMA. Wen et al., supra. T-cell supernatant was obtained by centrifugation of the cells and stored at -20ºC. The effectiveness of T-cell supernatants in enhancing proliferation of human B cells in EL4B5-B cell cultures was tested and the most effective supernatants were pooled and used in the experiments.

### Human T Cell Helper Assay for Antibody

Production by B Cells. 96-well tissue culture plates were coated with a 1:500 dilution of ascites fluid of anti-CD3 mAb CLB-T3/3 (CLB, Amsterdam, The Netherlands). As indicated costimulatory mAbs were added: anti CD2 mAbs CLB-T11.1/1 and CLB-T11.2/1 (CLB, Amsterdam, The Netherlands), both ascites 1:1000 and anti-CD28 mAb CLB-28/1 (CLB, Amsterdam, The Netherlands). Subsequently, tonsillar T cells (irradiated, 3000 Rad; 10⁵ per well), tonsillar B cells (10⁴ per well) and rIL-2 (20 U/ml) were added. The final volume of each cell culture was 200 µl. After 8 days, cells were spun down, and cell-free supernatant was harvested. The concentrations of human IgM and IgG in (diluted) samples were estimated by ELISA as described below.

### ELISA Assay for Immunoglobulin Quantification.

The concentrations of human IgM and IgG were estimated by ELISA. 96-well ELISA plates were coated with 4 µg/ml mouse anti-human IgG mAb MH 16-01 (CLB, Amsterdam, The Netherlands) or with 1.2 µg/ml mouse anti-human IgM mAD 4102 (Tago, Burlingame, CA) in 0.05 M carbonate buffer (pH = 9.6), by incubation for 16 h at 4°C. Plates were washed 3 times with PBS-0.05 % Tween-20 (PBS-Tween) and saturated with BSA for 1 hour. After 2 washes the plates were incubated for 1 h at 37°C with different dilutions of the test samples. After 3 washes, bound Ig was detected by incubation for 1 h at 37°C with 1 µg/ml peroxidase-labeled mouse anti-human IgG mAb MH 16-01 (CLB) or mouse anti-human IgM mAb MH 15-01 (CLB). Plates were washed 4 times and bound peroxidase activity was revealed by the addition of O-phenylenediamine as a substrate. Human standard serum (HOO, CLB) was used to establish a standard curve for each assay.

Flow Cytofluorometric Assay. ARC cells (10⁶ cells/sample) were incubated in 100 µl primary antibody (10 µg/ml in PBS-BSA or Hanks' balanced salt solution (HBSS) supplemented with 1% BSA and 0.05% sodium azide) for 20 min at 4°C. After 3 washes with PBS-BSA or HBSS-BSA, the cells were incubated in 100 µl FITC-labeled F(ab')₂ fragments of goat anti-(mouse IgG) antibodies (Jackson, West Grove, PA) for 20 min at 4°C. After 3 washes with PBS-BSA or HBSS-BSA and 1 wash with PBS, the cells were resuspended in 0.5 ml PBS. Analyses were performed with a FACSCAN V (Becton Dickinson, San Jose, CA).

Alternatively, EL4B5 cells were harvested before and at different time points during culture in medium containing PMA (5ng/ml) and human T-cell supernatant (5%). Cells were incubated for 30 minutes with 10 µl supernatant of transfected cells containing hCD40-Hµ diluted in 100 µl Hank's Balanced Salt Solution supplemented with 0.05% sodium azide (49c). This was followed by incubation with FITC-conjugated F(ab')₂ fragments of rabbit anti-(human IgM) (Central Laboratory of the Blood Transfusion service, Amsterdam, The Netherlands). As a control, cells were incubated with the FITC-conjugate only. For analysis a FACScan-4 cytofluorometer (Becton and Dickinson) was used. Non-vital cells were excluded from analysis by the use of propidium iodide.

### Example 1

### PCR Cloning of CD40 and B7

RNA was isolated from a population of EBV-transformed human spleen cells essentially as described by chirgwin et al. (1979). In brief, the cells were washed twice with phosphate buffered saline (PBS) and lysed in 5 M guanidinium thiocyanate in the presence of 0.7 M 2-mercaptoethanol. The cell lysate was layered on a discontinuous CsCl gradient (Chirgwin, *et al.*) and centrifuged for 16 hours at 26,000 rpm in a Beckman SW28 rotor. The RNA was recovered by dissolving the pellet in DEPC-treated H₂O. The RNA was precipitated with ethanol once, resuspended in DEPC treated H₂O, and stored at -70°C.

Total RNA (10 µg/reaction) was converted to cDNA using random hexamer priming in 50 µl reaction buffer containing 500 units LMV-RT (Bethesda Research Laboratories, Bethesda, MD), 5 µM random hexamers (Pharmacia, Piscataway, NJ), 1 mM DTT, dNTP mix (0.5 mM each), 10 mM Tris-HCL pH 8.3, 50 mM KCl, 2.5 mM MgCl₂ and 0.1 mg/ml BSA (bovine serum albumin). After incubation at 37°C for 1 hour, the samples were boiled for 3 min and stored at -70°C. The DNA encoding the CD40 and B7 molecules was generated by PCR using primers which contained sequences having homology to known CD40 and B7 sequence, where the primers also encoded restriction sites useful for cloning (Figure 2). These primers were based on the published cDNA coding sequences for B7 and CD40 (Freeman *et al.,* 1989; Stamenkovic *et al.,* 1989). All primers start with a C-G clamp at the 5' end followed by a restriction site for cloning (shown in bold, Figure 2). The underlined sequences in the backward primers, for the cloning of the soluble forms of B7 and CD40, represents an epitope recognized by a monoclonal antibody used for affinity purification. The numbers in brackets represent the location of the primers relative to the published cDNAs for CD40 and B7.

For PCR amplification, 1 µl of cDNA was mixed 1 µl (10 picomoles) of a forward primer, 1 µl (10 picomoles) of a backward primer, and 47 µl of PCR mix. The PCR mix consisted of 1.25 units Taq polymerase (Perkin-Elmer/Cetus, Norwalk, CT), dNTP mix (0.2 mM each), 10 mM Tris-cHL pH 8.3, 50 mM KCl, 2.5 mM MgCl₂ and 0.1 mg/ml BSA. The 50 µl of PCR mixture was overlaid with 70 µl mineral oil and subjected to 25 cycles of amplification in a Perkin-Elmer/Cetus thermocycler (denaturation at 95°C for 30 sec, primer annealing at 55°C for 30 sec and extension at 72°C for 1.5 min). PCR products were obtained after 25 amplification cycles.

The amplification products were digested with *BglII* and *KpnI* (Figure 1B) and isolated by size-fractionation. Before expression in baculovirus, the DNA sequence of each fragment was confirmed by sequencing analysis to prevent the introduction of PCR-induced mutations. The baculovirus transfer vector pAcC8 was also digested with *BglII* and *Kpnl* (Figure 1B). The amplified fragments were ligated to the linear pAcC8 vector (ratio of insert to vector was 3:1). The ligation products were transformed into bacterial strain DH5a (Gibco/BRL, Gaithersburg MD) and recombinant pAcC8 vectors were selected on the basis of ampicillin resistance. Recombinant plasmids were isolated from bacterial clones (Maniatis, et *al*.; Ausubel, et *al*.) and the presence of the insert of interest verified using polymerase chain reactions (see above). Large scale plasmid preparation was performed by standard procedures (Ausubel, *et al.*; Maniatis, *et al*.; Sambrook, *et al*.).

### Example 2

### Baculovirus Expression of Human CD40 and B7

Sequences encoding human CD40 and human B7 were recombined into the *Autographa californica* baculovirus (AcNPV) using the transfer vectors pAcCD40 (encoding the full-length CD40 molecule), pAcCD40-ED/Glu (encoding the extracellular domain of CD40), pAcB7 (encoding the full-length B7 molecule) and pCcB7-ED/Glu (encoding the cellular domain of the B7 molecule).

The plasmids were cotransfected with wild-type baculoviral DNA (2-10 pfu) (AcNPV; Summers et *al.*) into SF9 (*Spodoptera frugiperda*) cells at a density of 10⁶ cells/ml (Summers *et al.*)*.* Recombinant baculovirus-infected Sf9 cells were identified and clonally purified (Summers *et al.*).

For cell surface expression of recombinant proteins the cells were harvested after 48 hours of culture; for the production of secreted recombinant proteins, the cells were harvested after 72 hours of culture.

### Example 3

### Sf9 Cell ELISA

Sf9 insect cells infected with recombinant virus were cultured for 48 hours in 24-well plates. After removal of the tissue culture medium the plates were incubated for 45 min at room temperature (RT) with 0.25 ml of antibody in PBS with 1% BSA (PBS-BSA). After three washed with PBS-BSA, the plates were incubated for 35 min at RT with 250 µl of a 1/250 dilution of goat anti-(mouse total Ig) immunoglobulins conjugated to horseradish peroxidase (Zymed, South San Francisco, CA) in PBS-BSA. Unbound peroxidase activity was removed by washing five times with PBS-BSA. Bound peroxidase activity was revealed by the addition of an assay mixture prepared by diluting 0.5 ml of 2 mg/ml 3,3',5,5'-tetramethylbenzidine in ethanol to 10 ml with 10 mM Na acetate, 10 mM EDTA buffer (pH 5.0) and adding 0.03% (v/v) H₂O₂. The reaction was stopped after 10 min by adding 100 µl of 1 M H₂SO₄.

The above-described ELISA assays performed on live Sf9 cells gave the following results. Figure 3 presents the data for Sf9 cells infected with pAcB7 and pAcCD26 which were cultured for 48 hours in 24-well plates. The antibodies used in the ELISA were: B7-24, anti-(B7) (open bars), Ta-1, anti-(CD26) (hatched bars) and no primary antibody (gray bars).

Figure 4 presents the data for live Sf9 cells infected with pAcB7 and pAcCd40 which were cultured for 48 hours in 24-well plates. The antibodies used in the ELISA were: S2C6, anti-(CD40) (open bars) and no primary antibody (hatched bars).

### Example 4

### Fluorescent Cell Staining

### A. Fluorescent Cell Staining

Cells (10⁶/sample) were incubated in 10 µl primary antibody (10 µg/ml in PBS-BSA or HBSS (Hanks' Balanced Salt Solution, Gibco/BRL) supplemented with 1% BSA and 0.05% sodium azide) for 20 min at 4°C. After 3 washes with PBS-BSA or HBSS-BSA, the cells were incubated in 100 µl FITC-labeled Fab '2 fragments of goat anti-(mouse IgG)antibodies (Jackson, West Grove, PA) for 20 min at 4°C. After 3 washes with PBS-BSA or HBSS-BSA and 1 wash with PBS, the cells were resuspended in 0.5 ml PBS. Analyses were performed with a FACSSCAN V (Becton Dickinson, San Jose, CA).

General protocols for flow cytometric analysis and clinical data analysis for flow cytometry are detailed in Keren *et al.* and Coon *et al.* General blood cell counting techniques and DNA quantitation are described by Powers, Keren *et al.*, and Coon *et al.*

The data for fluorescent cell staining of ARC EBV transformed B cells is presented in Figure 5. In Figure 5A, the results for staining at 1:100 dilution of serum from a mouse immunized with B7 expressing Sf9 cells (solid line) or a 1:100 dilution of normal mouse serum (dotted line) are shown.

In Figure 5B, the results for staining with a 1:100 dilution of serum from a mouse immunized with CD40 expressing Sf9 cells (solid line) or a 1:100 dilution of normal mouse serum (dotted line) are shown.

In Figure 5C, the results for staining with a 1:100 dilution of serum from a mouse immunized with control Sf9 cells (solid line) or a 1:100 dilution of normal mouse serum (dotted line) are shown.

### B. Soluble Antigen Competition Assays

ARC EBV-transformed B cells were stained with anti-(B7) and anti-(CD40) monoclonal antibodies in the presence and absence of soluble B7 and soluble CD40. The antibodies and the soluble B7, soluble CD40 or controls were preincubated at RT for 20 min before addition to the ARC cells.

Figure 6A shows the results of staining with B7-24 (dotted line) or secondary antibody only (solid line). Figure 6B shows the results of staining with B7-24 alone (dotted line) or B7-24 preincubated with soluble B7 (solid line). Figure 6C shows the results of staining with B7-24 alone (dotted line) or B7-24 preincubated with soluble CD40. Figure 6D shows the results of staining with CD40-3A8 (dotted line) or second antibody alone (solid line). Figure 6E shows the results of staining with CD407A8 alone (dotted line) or CD403A8 preincubated with soluble B7 (solid line). Figure 6F shows the results of staining with CD403AB alone (dotted line) or preincubated with soluble CD40 (solid line).

### Example 5

### Host Animal Immunization

Female BALB/c mice were injected intraperitoneally at day 0 and day 14 with 5×10⁶ Sf9 cells infected with AcCD40 virus, AcB7 virus or AcCd3 virus (control virus). At day 21, 100 µl of serum was obtained to test for the presence of specific antibodies. After a rest period of at least two weeks, the mice received a final injection with 5×10⁶ cells infected with AcCD40 or AcB7 virus. Three days after this last injection, the spleen cells were used for cell fusion.

### Example 6

### Generation of Hybridoma Clones

Splenocytes from immunized BALB/c mice were fused with SP2/0 murine myeloma cells at a ratio of 10:1 using 50% polyethylene glycol as previously described by de Boer *et al.* (1988). The fused cells were resuspended in complete IMDM medium supplemented with hypoxanthine (0.1 mM), aminopterin (0.01 mM), thymidine (0.016 mM) and 0.5 ng/ml hIL-6 (Genzyme, Cambridge, MA). The fused cells were then distributed between the wells of 96-well tissue culture plates, so that each well contained 1 growing hybrid on average.

After 10-14 days the supernatants of the hybridoma populations were screened for specific antibody production. For the screening of specific antibody production by the hybridoma clones, the supernatants of 12 wells were pooled and used for fluorescent cell staining of EBV-transformed B cells as described in Example 4. Subsequently, the supernatants of the positive pools were tested individually. Positive hybridoma cells were cloned three times by limiting dilution in IMDM/FBS containing 0.5 ng/ml hIL-6. The results of the analysis are presented in Table 1.

**Table 1**

| Summary of Fusion Data for the Generation of Monoclonal Antibodies to Human CD40 and Human B7 | | |
|---|---|---|
| Fusion: | Anti-CD40 | Anti-B7 |
| No. of wells seeded after fusion | 480^{a} | 960 |
| No. of wells with hybridoma growth | 351 | 312 |
| No. of positive wells^{b} | 4 | 1 |
| Frequency of positive wells^{c} | 1.15 | 0.31 |

| | | |
|---|---|---|
| ^{a}Only half of the cells obtained after fusion were analyzed. ^{b}As determined by FACS analysis described in Example 4. ^{c}The frequency of positive wells is defined as the number of positive wells divided by the total number of wells with hybridoma growth, multiplied by 100. | | |

### Example 7

### Testing of Tonsillar B Cells

Tonsillar B lymphocytes were isolated from tonsils obtained from children undergoing tonsillectomy as described by deGroot *et al.* (1990). Briefly, the tissue was dispersed with scalpel blades, phagocytic cells and NK cells were depleted by treatment with 5 mM L-leucine methyl ester and T cells were removed by one cycle of rosetting with sheep erythrocytes treated with 2-aminoethyl isothiouronium bromide.

The anti-(CD40) and anti-(B7) monoclonal antibodies, were tested for their ability to bind to tonsillar B cells using the fluorescent cell staining assay described above in Example 4. For fluorescent stain analysis of cultured tonsillar B cells, propidium iodine was used to exclude dead cells.

Table 2 shows the results of the above analysis for the binding of anti-(CD40) monoclonal antibodies to highly enriched tonsillar B cells.

**Table 2**

| Binding of Anti-(CD40) Monoclonal Antibodies to Highly Enriched Tonsillar B Cells | | |
|---|---|---|
| Antibody | Specificity | % of Positive Cells^{a} |
| OKT3 | CD3 | 2.1 |
| LeuM3 | LeuM3 | 2.5 |
| B1 | CD20 | 88.0 |
| G28.5 | CD40 | 92.1 |
| CD40-5H7 | CD40 | 93.7 |
| CD40-SD12 | CD40 | 95.0 |
| CD40-3C6 | CD40 | 88.9 |
| CD40-3A8 | CD40 | 93.3 |

| | | |
|---|---|---|
| ^{a}The percentage of positive tonsillar cells was measured by fluorescent cell staining as described in Example 4. | | |

These data show that 89-95 percent of freshly isolated tonsillar B cells stained positive with the four anti-(CD40) monoclonal antibodies.

The reaction of the tonsillar B cells with monoclonal antibody G28.5 (Clark, *et al.*) were tested in essentially the same manner: about the same percentage of cells were positive with G28.5 as with the anti-(CD40) monoclonals of the present invention.

Table 3 shows the results of binding of anti-(B7) monoclonal antibody B-7-24 to highly enriched tonsillar B cells as determined by the fluorescent cell labelling described in Example 4.

**Table 3**

| Binding of Anti-(B7) Monoclonal Antibody B7-24 to Highly Enriched Tonsillar B Cells | | | |
|---|---|---|---|
| | | % of Positive Cells^{a} | |
| Antibody | Specificity | Donor 1 | Donor 2 |
| OKT3 | CD3 | 8.0 | 2.1 |
| B1 | CD20 | 74.0 | 88.0 |
| B7-24 | B7 | 12.6 | 16.8 |

| | | | |
|---|---|---|---|
| ^{a}The percentage of positive tonsillar cells was measured by fluorescent cell staining as described in Example 4. | | | |

These data show that 12-17 percent of freshly isolated tonsillar B cells stained positive with anti-(B7) monoclonal antibody B7-24.

### Example 8

### Blocking T Cell Proliferation with Mab B7-24

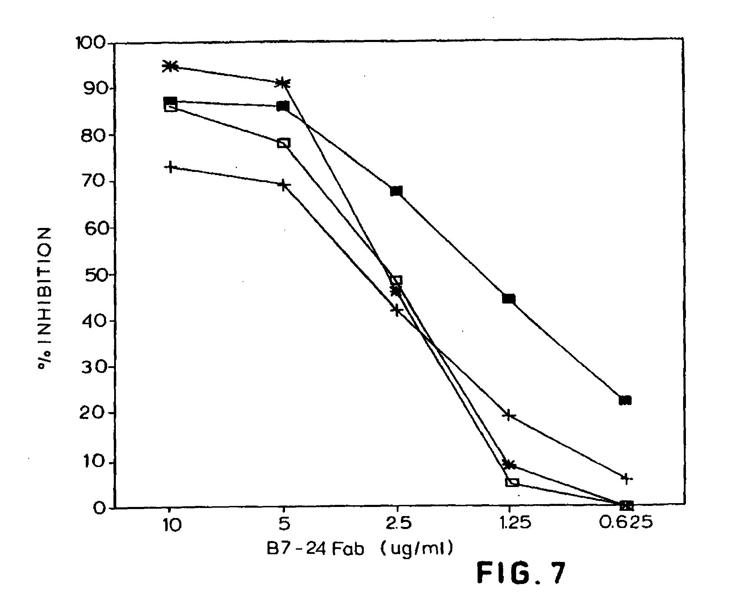

We studied the role of the B7 molecule in T cell activation using the T cell proliferation and MLC assays described above. Proliferation in the absence of B7-24 Fab fragments ranged from 20,000 to 60,000 cpm. Figure 7 shows that this Mab binds to a functionally important domain of the B7 molecule, since it can completely block anti-CD3-induced, B7-mediated induction of T cell proliferation. Data shown are the ± S.D. of 4 individual experiments using T cells of different donors.

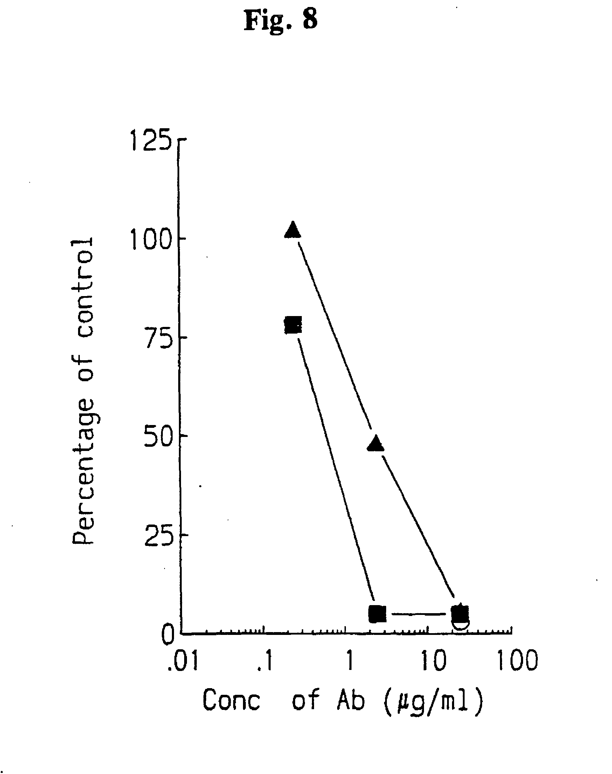

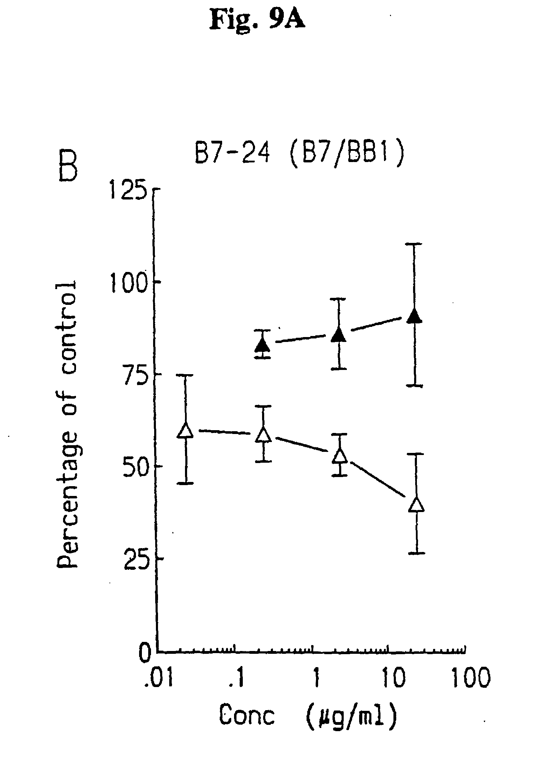

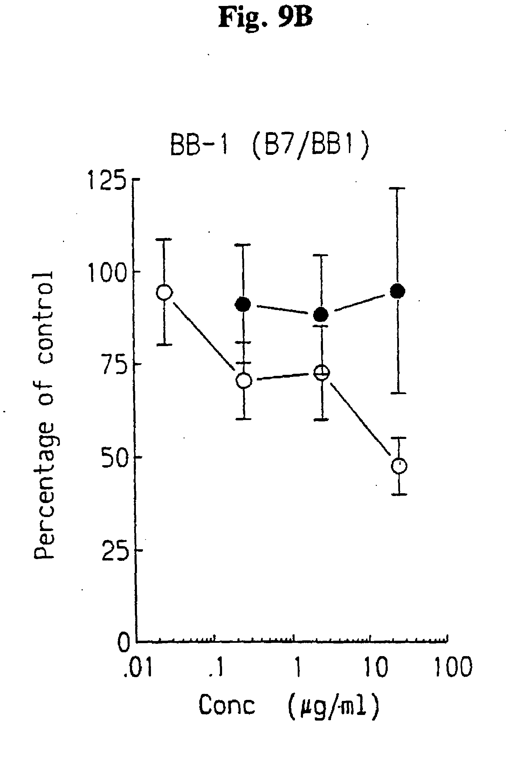

Figure 8 shows the inhibition of anti-CD3-induced, B7-mediated proliferation of T cells by anti-B7 Mabs B7-24 (squares) or BB-1 (triangles). It was therefore surprising to find that Mab B7-24 could not inhibit T cell activation in primary MLC using B7-positive EBV-transformed B cells as stimulator cells. Figure 9 shows the effect of blocking B7/CD28 interaction during primary MLC (closed symbols) and secondary MLC (open symbols). Purified T cells were stimulated with the EBV-transformed B cell line ARC for 3 days in the presence or absence of different concentrations of Mab B7-24 (Figure 9A) or BB-1 (Figure 9B). Proliferation in the absence of antibody ranged from 15,000 to 30,000 in primary MLC and from 20,000 to 60,000 in secondary MLC. Data shown are the mean ± S.D. of 4 individual experiments using T cells from different donors.

From this experiment, it is clear that anti-B7 monoclonal antibodies cannot completely block primary MLCs. Under the same experimental conditions, however, a Mab to CD3 could almost completely block the activation of T cells in the primary MLC. Interestingly, when Mab B7-24 was tested for its inhibitory capacity in secondary mixed lymphocyte cultures using pre-activated T cells, it was able to inhibit the activation of T cells.

### Example 9

### Blocking T Cell Proliferation with cyclosporin A and/or Mab B7-24

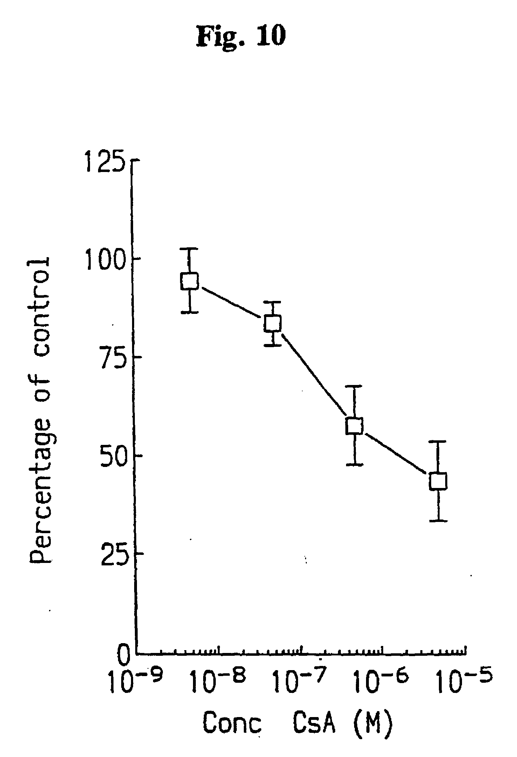

We used the T cell proliferation assay described above to determine whether the costimulation of T cells with B7 is also resistant to inhibition with CsA. Figure 10 shows that when T cells are induced to proliferate with anti-CD3 Mab, costimulation with B7 can be inhibited in a dose-dependent manner by CsA. However, it was not possible to completely block the activation of T cells with CsA concentrations that are not toxic. When CsA was used to block primary MLC (results not shown) or secondary MLC (see Table 4), similar results were obtained. These experiments clearly suggest that B7-CD28/CTLA4 mediated T cell proliferation is only partially sensitive to the inhibitory action of CsA. This lack of complete inhibition when T cells are costimulated with B7 in vitro could mimic what happens *in vivo* during graft rejection or acute GVHD despite treatment with CsA.

**Table 4**

| CsA and Mab B7-24 Svnerqy in blocking T-cell proliferation in Secondary MLC* | | | | | | | |
|---|---|---|---|---|---|---|---|
| B7-24 (µg/ml) | Cyclosporin A concentration | | | | | | |
| | 6x10⁻⁶M | 3x10⁻⁶M | 10⁻⁶M | 6x10⁻⁷M | 3x10⁻⁷M | 10⁻⁷M | None |
| 2.5 | 971 | 609 | 601 | 790 | 697 | 1,159 | 25,209 |
| 0.25 | 553 | 545 | 601 | 788 | 559 | 882 | 20,753 |
| 0.025 | 897 | 939 | 1,121 | 1,592 | 1,570 | 2,818 | 29,364 |
| None | 12,687 | 15,593 | 23,484 | 24,589 | 27,629 | 33,235 | 62,598 |

| | | | | | | | |
|---|---|---|---|---|---|---|---|
| *Table entries reflect T-cell proliferation in CPM. Proliferation was measured using the T-cell proliferation assay described above. Data shown for one of four experiments. | | | | | | | |

Table 4 shows that CsA alone or mAb B7-24 alone gave a dose-dependent, but incomplete, inhibition of T cell activation. However, when CsA and B7-24 were combined, T cell activation was completely blocked. Interestingly, addition of 0.025 µg/ml B7-24 gave almost the same amount of blocking as 2.5 µg/ml. Furthermore, in the presence of 0.025 µg/ml B7-24, decreasing the CsA concentration 60-fold still resulted in more than 90% inhibition of T cell activation, being more than the maximal inhibition with the highest CsA concentration alone.

This synergy between Mab B7-24 and CsA was specific for the B7/CD28 interaction, since it was not observed using Mabs to either ICAM-1 or CD72 in the same proliferation assay, as shown in Table 5.

**Table 5**

| Effects of CsA with B7, ICAM-1 or CD72 Mabs in blocking T-cell proliferation in a secondary MLC | | | | | |
|---|---|---|---|---|---|
| Monoclonal Antibody | Concentration (µg/ml) | Cyclosporin A Concentration | | | |
| | | 6 x 10⁻⁷ M | 3 x 10⁻⁷ M | 10⁻⁷ M | None |
| B7-24 | 2.5 | 3 | 3 | 3 | 40 |
| | 0.25 | 3 | 2 | 3 | 33 |
| (α-B7) | 0.025 | 6 | 6 | 6 | 47 |
| 84H10 | 2.5 | 76 | 84 | 81 | 115 |
| | 0.25 | 80 | 80 | 76 | 99 |
| (α-ICAM-1) | 0.025 | 91 | 99 | 96 | 99 |
| WL225 | 2.5 | 106 | 107 | 89 | 98 |
| | 0.25 | 108 | 106 | 91 | 100 |
| (α-CD72) | 0.025 | 109 | 105 | 93 | 106 |

| | | | | | |
|---|---|---|---|---|---|
| *Table entries reflect T-cell proliferation in CPM. Proliferation was measured using the T-cell proliferation assay described above. | | | | | |

### Example 10

### Blocking T Cell Proliferation with Mab B7-24 and Other Immunosuppressive Agents

The protocol of Example 9 above is used to show the blocking of T cell proliferation using Mab B7-24 in conjunction with other immunosuppressive agents. The proliferation assay protocol in Example 9 is followed substituting (A) FK506, (B) rapamycin, (C) methotrexate, (D) prednisolone, or (E) dexamethasone for CsA.

### Example 11

### Blocking Cytotoxic T Lymphocyte Activity with Cyclosporin A and/or Mab B7-24

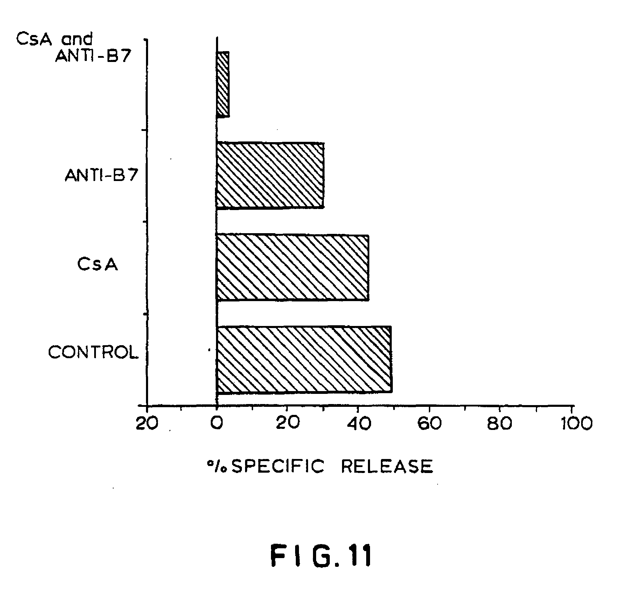

We used the cytotoxicity assay described above to test whether CsA and B7-24 could also cooperate in blocking induction of CTL activity in alloantigen-specific activation of T cells. Purified T cells were stimulated with the EBV-transformed B cell line ARC for 6 days, followed by a 2-day culture period in medium alone. CTL activity of the T cells was analyzed after restimulation for 3 days with the EBV-transformed B cell line ARC in the presence of medium alone; 400 µg/ml CsA; 10 µg/ml Mab B7-24; or 400 µg/ml CsA and 10 µg/ml Mab B7-24, as shown in Figure 11. T cells activated by the alloantigen in these cultures were efficiently induced to become cytolytic, since about 50% of the ARC target cells could be lysed in the 4 hour assay. This induction of CTL activity in secondary MLC could only be slightly inhibited with 400 ng/ml CsA. Addition of 10 µg/ml Mab B7-24 during the secondary MLC resulted in about 40% inhibition. However, combining CsA and Mab B7-24 resulted in almost complete blockage of the CTL activation.

### Example 12

### Blocking Cytotoxic T Lymphocyte Activity with Mab B7-24 and other Immunosuppressive Agents

The protocol of Example11 above is used to show the blocking of cytotoxic T lymphocyte activity using Mab B7-24 in conjunction with other immunosuppressive agents. The cytotoxicity assay protocol in Example 11 is followed substituting (A) FK506, (B) rapamycin, (C) methotrexate, (D) prednisolone, or (E) dexamethasone for CsA.

### Example 13

### Combining CsA and Mab B7-24 During Alloantigen-Specific T-Cell Activation

We investigated whether T cells stimulated in a primary MLC assay (as described above) with the alloantigen in the presence of CsA and Mab B7-24 could respond to secondary antigen stimulation. Purified T cells were stimulated with the EBV-transformed B cell line ARC for 6 days in the presence or absence of 10 µg/ml Mab B7-24 and 400 µg/ml CsA, followed by a 2 day culture period in medium alone. CTL activity of the T cells was analyzed after re-stimulation for 3 days with the EBV-transformed B cell line ARC. The data in Table 6 is that of a representative experiment, showing that 6-day exposure to the alloantigen in the presence of both CsA and Mab B7-24, but not in the presence of CsA or Mab B7-24 alone, resulted in total unresponsiveness for subsequent challenge with the alloantigen in secondary MLC. This unresponsiveness was not due to lack of viability of the cells after the primary MLC, since in a control experiment the cell population could still be induced to become cytotoxic after stimulation with immobilized anti-CD3 Mab or cells expressing a non-related alloantigen (results not shown).

**Table 6**

| Alloantigen-Specific T Cell Tolerance Induced by the combination of CsA and B7-24 | | |
|---|---|---|
| Additions of Culture Medium During Primary MLC* | (% specific release) | |
| | P815 | ARC |
| None | 80 | 49 |
| B7-24 (10 µg/ml) | 78 | 24 |
| CsA (400 ng/ml) | 76 | 37 |
| B7-24 & CsA | 0 | 0 |

| | | |
|---|---|---|
| * CTL activity of purified T cells in secondary MLC with ARC cells as stimulator cells, after primary MLC as described above. CTL activity measured in T cell cytotoxicity assay as described above. | | |

### Example 14

### Combining Mab B7-24 and an Immunosuppressive Agent During Alloantigen-Specific T-Cell Activation

The protocol of Example 13 above is used to show the ability of Mab B7-24 in conjunction with other immunosuppressive agents to block secondary antigen stimulation. The CTL assay protocol in Example 13 is followed substituting (A) FK506, (B) rapamycin, (C) methotrexate, (D) prednisolone, or **(E) dexamethasone for CsA.**

### Example 15

### Costimulation of B-Cell Proliferation Using Anti-CD40 mAbs

Four hybridomas producing monoclonal antibodies against human CD40 were generated as described in Examples 1-7. These mAbs were shown to bind to a similar proportion of tonsillar B cells as anti-CD40 mAb G28.5 does. de Boer et al. J. Immunol. Methods (1992) 152:15. Three of these monoclonal antiodies (5D12, 3A8 and 3C6) which were of the IgG2b subclass, were tested for their ability to deliver activation signals to human B cells in the B-cell proliferation assay described above.

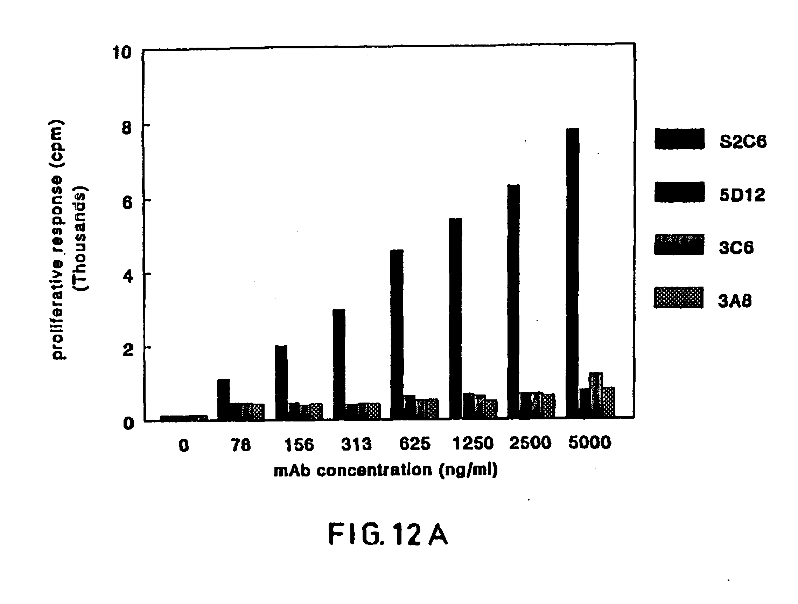

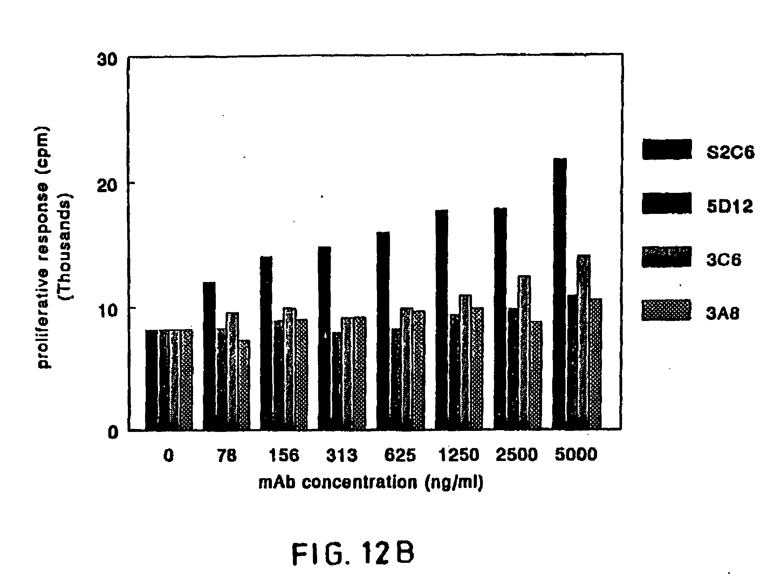

Human tonsillar B cells (4 x 10⁴ per well) were cultured in 200 µl in microwells in the presence of anti-IgM coupled to Sepharose beads (5 µ/ml) (Figure 12A) or in the presence of anti-IgM plus rIL-2 (100 U/ml) (Figure 12B). Varying concentrations of the anti-CD40 mAbs S2C6, 5D12, 3C6 or 3A8 were added and [³H]thymidine incorporation was measured at day 3 after 18 h pulsing. Data presented in Figure 12A are means derived from experiments with B-cell preparations from three different donors with duplicate incubations. Data of Figure 12B are means of duplicate incubations from one experiment out of two with comparable results.

None of the novel anti-CD40 mAbs was able to significantly costimulate human B-cell proliferation in the presence of immobilized anti-IgM or in the presence of immobilized anti-IgM and IL-2. In contrast, anti-CD40 mAb S2C6 costimulated human B-cell proliferation in a concentration dependent fashion.

### Example 16

### Induction of B-Cell Proliferation Using Anti-CD40 mAbs

The mAbs tested in Example 15 were tested for their ability to induce proliferation of human B cells in the Banchereau-like Assay described above, i.e., by presenting the anti-CD40 mAb on adherent cells expressing FcγRII. As antibody presenting cells, mouse 3T6 transfectant cells expressing the HR allellic form of human FcγRII were used. It was observed that anti-CD40 mAb S2C6 together with IL-4 induced substantial proliferation of tonsillar human B cells in this system, as assessed by measurement of [³H]thymidine incorporation. Anti-CD40 mAbs 5D12, 3C6 or 3A8 however, did not induce proliferation of human B cells in this culture system (data not shown).

### Example 17

### Inhibition of S2C6 stimulated B-Cell Proliferation Using Anti-CD40 mAbs

The anti-CD40 mAbs were also tested for their ability to inhibit the costimulation of human B-cell proliferation by anti-CD40 mAb S2C6 using the B-cell Proliferation Assay described above. Human tonsillar B cells (4 x 10⁴ per well) were cultured in 200 µl in microwells in the presence of anti-IgM coupled to Sepharose beads (5 µg/ml) and anti-CD40 mAb S2C6 (1.25 µg/ml). Varying concentrations of anti-CD40 mAbs 5D12, 3C6 or 3A8 were added and [³H]thymidine incorporation was assessed after 3 days. As a control anti-(glucocerebrosidase) mAb 8E4 was added in similar concentrations. Barneveld et al. Eur. J. Biochem. (1983) 134:585. Data are means ± S.D. derived from experiments with B cells from two different donors with duplicate incubations.

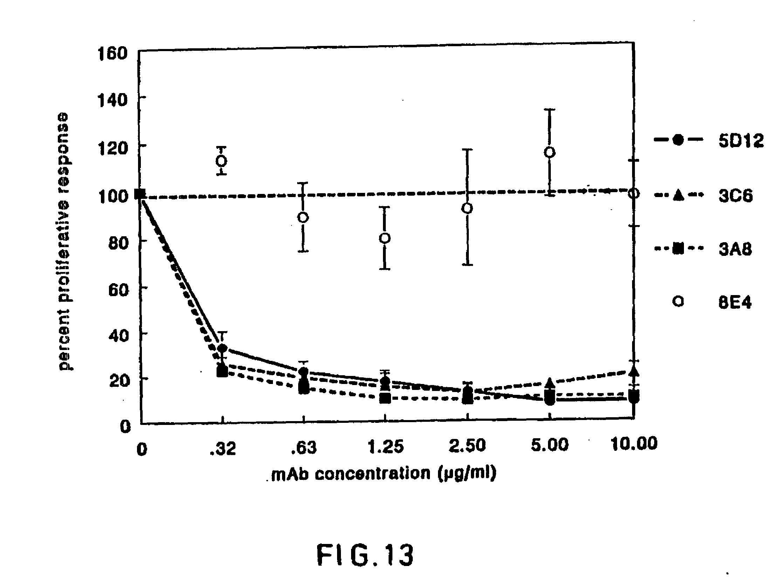

It was found that each of the anti-CD40 mAbs 5D12, 3A8 and 3C6 could inhibit the costimulation of anti-IgM induced human B-cell proliferation by mAb S2C6 (Figure 13). In contrast, no significant inhibition was seen with equivalent amounts of non-relevant mAb 8E4, directed to β-glucocerebrosidase. Barneveld et al., supra. Thus, it was concluded that these anti-CD40 mAbs do not deliver stimulatory signals to the proliferation of human B cells, but, conversely, can inhibit stimulatory signals exerted by triggering anti-CD40 with another mAb. Therefore, these mAbs were considered to be excellent tools to investigate whether signaling via CD40 plays a role in the stimulation of human B-cell proliferation by EL4B5 cells.

### Example 18

### Effects of Anti-CD40 mAbs on EL4B5-Induced Human B-Cell Proliferation

The effect of anti-CD40 mAbs on EL4B5-induced human B-cell proliferation was tested using the B-cell Activation Assay described above. Human tonsillar B cells (1000 per well) were cultured together with irradiated EL4B5 cells (50,000 per well) in the presence of 5% supernatant of activated human T cells and 5 ng/ml PMA. Anti-CD40 mAbs 5D12, 3C6 or 3A8 were added in varying concentrations. As a control, mAb MOPC-141 (IgG2b) was added. After six days of culture, [³H]thymidine incorporation was assessed.

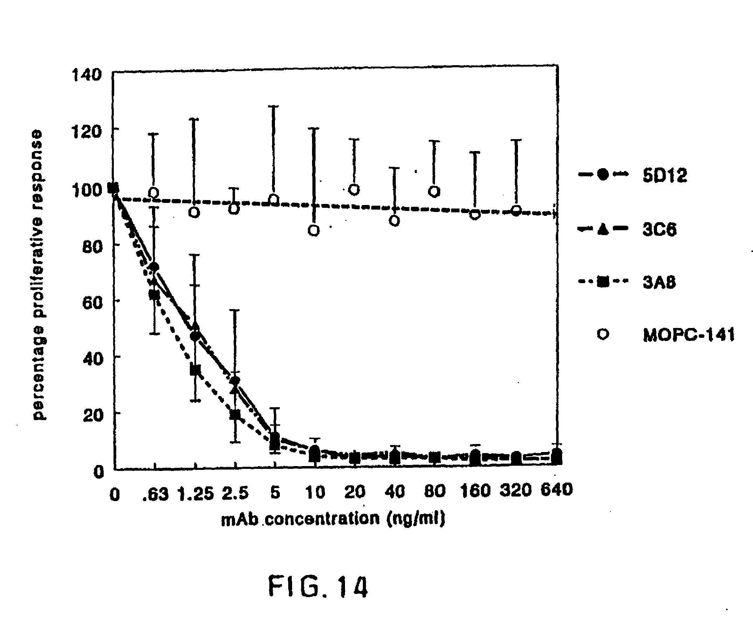

Figure 14 shows that addition of anti-CD40 mAbs 5D12, 3C6 or 3A8 resulted in a concentration-dependent inhibition of human B-cell proliferation. Data are means ±S.D. derived from experiments with B cells from four different donors with duplicate incubations. [³H]-thymidine incorporation values found for incubations without mAb were (means ± S.D.) 10460 ± 1843 cpm, 6982 ± 1729 cpm, 4362 ± 1020 cpm and 1543 ± 3190 in the four different experiments, respectively. [³H]-thymidine incorporation in B cells alone amounted to 40 ± 5 cpm and in irradiated EL4B5 cells alone 31 ± 15 cpm.

Very potent inhibition occurred. At concentrations as low as 10 ng/ml each, the three anti-CD40 mAbs 5D12, 3C6 and 3A8 inhibited human B-cell proliferation completely. Half-maximal inhibition was found at about 1 ng/ml. In contrast, isotype matched IgG2b mouse myeloma protein MOPC-141 had no significant effect on [³H]-thymidine incorporation. Similar inhibition was observed when [³H]-thymidine incorporation was assessed at day 4 of the culture instead of day 6, thus excluding the possibility that the observed effect was due to a change in the kinetics of the proliferation under influence of the anti-CD40 mAb (data not shown).

For comparison, the influence of a few mAb directed against other B-cell surface structures was investigated. Neither anti-CD20 mAb B1 or anti-B7 mAb B7-24 (the latter mAb was generated by a procedure similar to that used for generating the anti-CD40 mAb used in Figure 14, in concentrations similar to those used in the experiments with the anti-CD40 mAb, had any effect on EL4B5-induced human B-cell proliferation (data not shown). Therefore, it may be concluded that the inhibitory effect of anti-CD40 mAb on EL4B5-induced B-cell proliferation is not due to masking of the B-cell surface.

### Example 19

### Effects of hCD40.Hµ on EL4B5-Induced Human B-Cell Proliferation

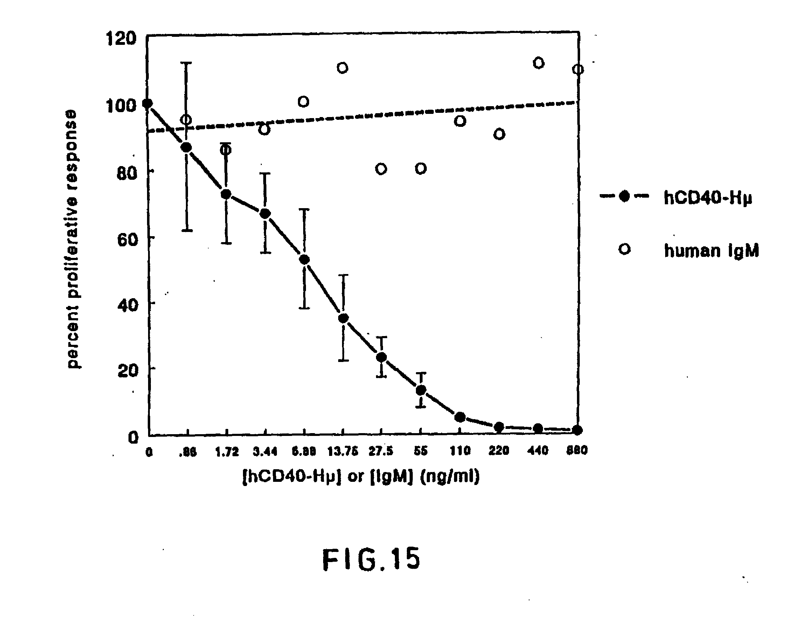

In order to investigate whether EL4B5 cells expressed a membrane structure which binds CD40, a fusion protein consisting of the extracellular domain of CD40 and human IgM constant domains CH₂, CH₃ and CH₄ (hCD40.Hµ) was used for flow fluorocytometric analysis. Lane et al., supra. Non-activated EL4B5 cells did not bind the fusion protein. However, upon culturing EL4B5 cells together with PMA (5 ng/ml) and 5% human T-cell supernatant, which are the conditions needed for activation of human B cells, a low binding of hCD40.Hµ was found (data not shown). This small shift in fluorescence was found consistently in three independent experiments. The minimal activation period needed for induction of the CD40 binding was 24 hours. To determine whether binding of hCD40.Hµ to the EL4B5 cells would inhibit EL4B5-induced human B-cell proliferation like anti-CD40 mAb did, the fusion protein was titrated into cocultures of EL4B5 cells with human B cells using the B-cell Activation Assay described above. Figure 15 shows that the fusion protein did indeed inhibit [³H]-thymidine incorporation in a concentration-dependent manner and, like the anti-CD40 mAb used in the experiments shown in Figure 14, was able to inhibit B-cell proliferation induced by the EL4B5 cells completely.

### Example 20

### Effects of Anti-CD40 mAbs on Human T-Cell-Induced Antibody Production by Human B-Cells

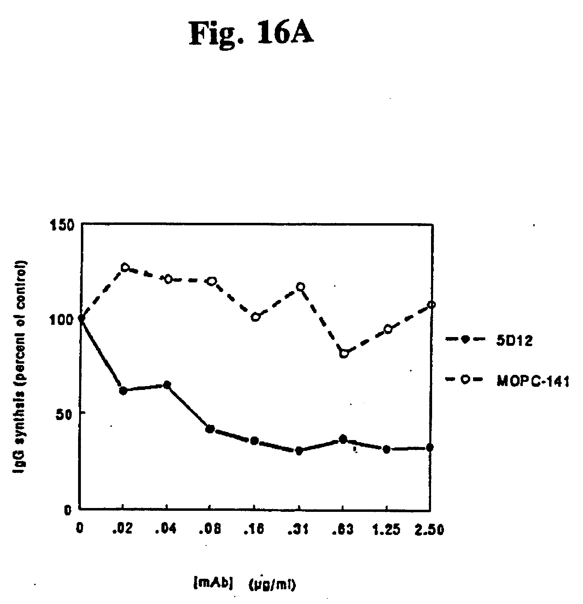

The antibodies were also tested for their capacity to inhibit immunoglobulin production by B cells, stimulated in a contact-dependent manner with activated T cells using the T-cell helper assay described above. Human tonsillar B cells (10⁴/well) were cultured together with irradiated purified T cells (3000 rad, 10⁵ /well) in 96-well plates, coated with anti-CD3 mAb and with or without different nAbs to costimulate the T cells. After 8 days of culture the supernatants were harvested for the determination of immunoglobulin production by the B cells. Immunoglobulin production by the B cells was assessed by the ELISA assay described above. Anti-CD40 mAb 5D12 was added in varying concentrations from the onset of the cultures. As a control, mAb MOPC-141 was added. Figure 16A shows that when T cells were stimulated with immobilized anti-CD3 mAb and costimulated with soluble anti-CD2 and anti-CD28 mAbs, addition of anti-CD40 mAb 5D12 resulted in a concentration dependent inhibition of IgG production by human B cells. IgM production by the B cells was inhibited to the same extent. Similar results were obtained with the anti-CD40 mAbs 3C6 and 3A8 and with the hCD40.Hµ fusion protein.

The anti-CD40 mAbs of this invention exhibited very potent inhibition. At concentrations as low as approximately 30 ng/ml, each of the three anti-CD40 mAbs gave 50% of maximal inhibition. In contrast, the isotype-matched IgG2b mouse myeloma protein MOPC-141 had no effect on the immunoglobulin production.

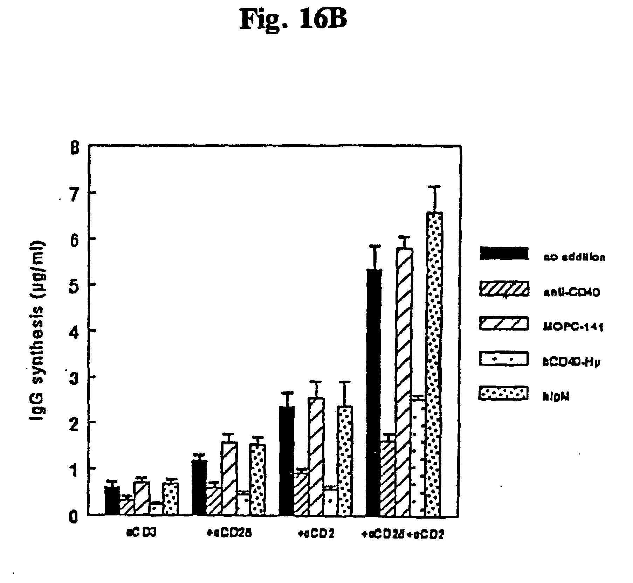

The inhibitory effect by the three anti-CD40 mAbs was not specific for the manner of activation of the T cells providing the CD40 ligand helper activity. Figure 16B shows that under all the T-cell stimulation conditions (anti-CD3 alone; anti-CD3 + anti-CD2; anti-CD3 + anti-CD28; and anti-CD3 + anti-CD2 + anti-CD28), addition of the anti-CD40 mAb 5D12 results in strong inhibition of immunoglobulin production by the human B cells. The inhibition is comparable to the amount of inhibition with the hCD40.Hµ fusion protein, known to completely block the CD40-CD40 ligand interaction. The percentage of inhibition varied from 40 to 70% depending on the T-cell activation conditions. In contrast, the isotype-matched IgG2b mouse myeloma protein MOPC-141, or human IgM (as control for the hCD40.Hµ fusion protein) had no effect on immunoglobulin production by the human B cells.

### Deposition of Cultures

The hybridomas used in the above examples were deposited in and accepted by the American Type Culture Collection (ATCC), 12301 Parklawn Drive, Rockville, Maryland, USA, under the terms of the Budapest Treaty. This deposition does not indicate that these hybridomas are necessary to practice the invention described above or claimed below.

| Hybridoma | Deposit Date | Accession No. |
|---|---|---|
| B7-24 | May 6, 1993 | HB 11341 |
| 3C6 | May 6, 1993 | HB 11340 |
| 5D12 | May 6, 1993 | HB 11339 |

The present invention has been described with reference to specific embodiments. However, this application is intended to cover those changes and substitutions which may be made by those skilled in the art without departing from the spirit and the scope of the appended claims.

### References

Ausubel, F. M. et al. Current Protocols in Molecular Biology, John Wiley and Sons, Media, PA.
Bohinski, R.C., Modern Concepts in Biochemistry, Second Edition, Allyn and Bacon, Inc.
Carroll, W.P., Thielemans, K., Dilley, J., and Levy, R. (1986) J. immunol. Methods 89: 61.
Chirgwin, J.M., et al., Biochemistry 17:5294 (1979). Clark, E.A., et al., Proc. Natl. Acad. Sci. U.S.A. 83:4494 (1986).
Coon, J.S., et al. (editors), Diagnostic Flow Cytometry, Academy of Pathology Inc. (1991).
Crea, R., U. S. Patent No. 4,888,286, issued December 19, 1989.
de Boer, M., et al., J. Immunol. Methods 113:143 (1988).
DeGroot, C., et al., Lymphokine Research 9:321 (1990).
DiSanto, J.P., et al., J. Immunol. Methods 141:123 (1991).
Eaton, M. A. W., *et al.,* U. S. Patent No. 4,719,180, issued Jan. 12, 1988.
Freedman, A.S., et al., J. Immunol. 139:3260 (1987). Freeman, G.J., et al., J. Immunol. 143:2714 (1989). Harlow, E., et al., Antibodies: A Laboratory Manual, Cold Spring Harbor Laboratory Press (1988). Huynh, T.V., et al., in "DNA Cloning, Volume 1," ed. D.M.Glover, Washington, D.C.: IRL Press, 1985 (Chapter 2). Keren, D.F., (editor), Flow Cytometry in Clinical Diagnosis, American Society of Clinical Pathologists (1989).
Lackuow, V.A., et al., "Cloning and express ion of heterologous genes in insect cells with baculovirus vectors", in Recombinant DNA Technology and Applications (C. Ho., A. Prokop, and R. Bajpai, eds.) McGraw-Hill, NY (19991).
Luckow, V.A., et al., Virology 170:31 (1989). Maiorella, B., et al., Biotechnology 6:1406 (1988). Maniatis, T., et al., Molecular Cloning: A Laboratory Manual, Cold Spring Harbor Laboratories, Cold Spring Harbor, NY (1982).
Matsuura, Y., et al., J. Gen. Vir. 68(pt.5):1233-1250 (1987).
Miller, L.K. (1988) in Vectors: A Survey of Molecular Cloning Vectors and Their Uses, Rodriguez, R.L. and Denhardt, D.T., eds. Butterworths, Boston. Mishell, B. B. and Shiigi, S. M., eds. (1980) Selected Methods in Cellular Immunology, W.H. Freeman and Co., San Francisco.
Mullis, K.B., U. S. Patent No. 4,683,202, issued 28 July 1987.
Mullis, K.B., *et al.,* U. S. Patent No. 4,683,195, issued 28 July 1987.
Nyunoya, H., et al., AIDS Res. Hum. Retrovir. 6(11):1311-1321 (1990).
Paulie, S., et al., Cancer Immunol. Immunother. 20:23 (1985).
Perkins, S. et al (1989) in Borrebaeck, C.A.K., Hagen, I. (eds) Electromanipulation in Hybridoma Technology, A Laboratory Manual, Stockton Press, New York.
Perkins, S., Zimmerman, U., Foung, S.K.H. (1991) Hum. Antibod. Hybridomas 2: 155-159.
Powers, L.W., Diagnostic Hematology: Clinical and Technical Principles, C.V. Mosby Company (1989). Sambrook, J., et al., In Molecular Cloning: A Labo-ratory Manual, Cold Spring Harbor Laboratory Press, Vol. 2 (1989).
Saiki, R.K., et al., Science 230:1350 (1985).
Saiki, R.K., et al., Science 239:487 (1988).
Sekine, H., et al., Gene 65(2):187-193 (1988).
Stamenkovic, I., at al., EMBO J. 8:1403 (1989).
Summers, M.D., et al., A manual of Methods for Baculovirus Vectors and Insect Cell Culture Procedures, Texas Agricultural Experimental Station Bulletin, No. 1555 (1987).
Takehara, K., et al., J. Gen. Virol. 69(pt.11):2763-2777 (1988).
Valle, A., et al., Immunology 69:531 (1990).
Webb, N.R., et al., Technique 2:173 (1990).
Webb, N.R., et al., Proc. Natl. Acad. Sci. U.S.A. 86:7731 (1989).
Wu, A.Z., et al., Chin. J. Biotech. 6(4):237-242 (1990).
Yokochi, T., et al., J. Immunol. 128:823 (1982). Yoshio, T., et al., U. S. Patent No. 4,849,350, issued July 18, 1989.

## Claims

1. A method for preventing or treating an antibody-mediated disease in a patient, the method comprising administering to a patient in need of such treatment a therapeutically effective amount of a monoclonal antibody capable of binding to a human CD40 antigen located on the surface of a human B cell, wherein the binding of the antibody to the CD40 antigen prevents the growth or differentiation of the B cell, in a pharmaceutically acceptable excipient.

2. The method of claim 1, wherein the antibody-mediated disease is selected from the group consisting of IgE-mediated disease, sytematic lupus erythematosus (SLE), primary biliary cirrhosis (PBC), and idiopathic thrombocytopenic purpura (ITP).

3. The method of claims 1 or 2 wherein the monoclonal antibody is selected from the group consisting of 5D12, 3A8 and 3C6.

4. A CD40 antigen epitope immunoreactive with an anti-CD40 monoclonal antibody wherein the binding of said monoclonal antibody to a human CD40 antigen located on the surface of a human B cell prevents the growth or differentiation of the B cell.

5. Monoclonal antibody B7-24.

6. A monoclonal antibody capable of binding to a human CD40 antigen located on the surface of a human B cell, wherein the binding of the antibody to the CD40 antigen prevents the growth or differentiation of the B cell.

7. The monoclonal antibody of claim 6 selected from the group consisting of 5D12, 3A8 and 3C6.

8. A method of generating an immortalized cell line producing a monoclonal antibody specific against a selected membrane-associated antigen, comprising
producing the membrane-associated antigen on the cell surface of insect cells,
injecting said insect cells into a host animal,
recovering from the host animal cells capable of producing antibody molecules,
immortalizing such cells for growth in cell culture,
screening the immortalized cells for production of antibodies specific to the membrane-associated antigen in a binding assay employing non-insect cells having the membrane-associated antigen on the surface of the non-insect cells, and
selecting those immortalized cells producing antibodies which bind to the membrane-associated antigen on the surface of said non-insect cells.

9. The method of claim 8, wherein said producing further comprises
inserting DNA sequence encoding the membrane-associated antigen operatively into a baculoviral vector suitable for expression of said antigen in selected insect cells,
transfecting insect cells with the vector, and
selecting insect cells transformed with the vector which produce the membrane-associated antigen on the cell surface of the insect cells.

10. A method of producing sera containing antibodies specific against a selected membrane-associated antigen, comprising
producing the membrane-associated antigen on the cell surface of insect cells,
injecting said insect cells into a host animal, and
screening sera from said animal for production of antibodies specific to the membrane-associated antigen in a binding assay employing non-insect cells having the membrane-associated antigen on the cell surface.

11. A method for preventing transplant rejection in a patient, the method comprising administering to a patieint in need of such treatment a therapeutically effective amount of (a) a molecule that binds to the B7 antigen; and (b) an immunosuppressive agent in a pharmaceutically acceptable excipient.

12. A method for preventing or treating graft versus host disease (GVHD) in a patient, the method comprising administering to a patient in need of such treatment of therapeutically effective amount of (a) a molecule that binds to the B7 antigen; and (b) an immunosuppressive agent in a pharmaceutically acceptable excipient.

13. A method for preventing or treating rheumatoid arthritis in a patient, the method comprising administering to a patient in need of such treatment a therapeutically effective amount of (a) a molecule that binds to the B7 antigen; and (b) an immunosuppressive agent in a pharmaceutically acceptable excipient.

14. The method of claims 11, 12 or 13, wherein the molecule that binds to the B7 antigen is an anti-B7 antibody.

15. The method of claims 11, 12, 13 or 14, wherein the immunosuppressive agent is selected from the group consisting of cyclosporin A, FK506, rapamycin and corticosteroids.


SEQUENCE LISTING

(1) GENERAL INFORMATION:
(i) APPLICANT: de Boer, Mark
Conroy, Leah B.

(ii) TITLE OF INVENTION: A Method for Generation of Antibodies to
Cell Surface Molecules

(iii) NUMBER OF SEQUENCES: 8

(iv) CORRESPONDENCE ADDRESS:
(A) ADDRESSEE: Cetus Oncology Corporation
(B) STREET: 4560 Horton Street, R-440
(C) CITY: Emeryville
(D) STATE: CA
(E) COUNTRY: USA
(F) ZIP: 94608

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE: 09 JULY 1993
(C) CLASSIFICATION:

(viii) ATTORNEY/AGENT INFORMATION:
(A) NAME: McGarrigle, Philip L.
(B) REGISTRATION NUMBER: 31,395
(C) REFERENCE/DOCKET NUMBER: 0925.100

(ix) TELECOMMUNICATION INFORMATION:
(A) TELEPHONE: (510) 601-2718
(B) TELEFAX: (510) 655-3542

(2) INFORMATION FOR SEQ ID NO:1:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

GCGCTGCAGC ATCTGAAGCC ATGGGCC 27

(2) INFORMATION FOR SEQ ID NO:2:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

CGCGGTACCT TGCTTCTGCG GACACTG 27

(2) INFORMATION FOR SEQ ID NO:3:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:3:

GCGCTGCAGC ATCTGAAGCC ATGGGCC 27

(2) INFORMATION FOR SEQ ID NO:4:

(i) SEQUENCE CHARACTERISTICS:
(A) LENGTH: 61 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:4:

GCGCGGTACC TTACTCCATG GGCATGTATT CCTCTTCCTC GTTATCAGGA AAATGCTGTT G 61

(2) INFORMATION FOR SEQ ID NO:5:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

GCGTAGATCT GGTCTCACCT CGCCATGGTT CG 32

(2) INFORMATION FOR SEQ ID NO:6:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:6:

GCGTGGTACC CCACACTCCT GGGTGGGTGC AGCC 34

(2) INFORMATION FOR SEQ ID NO:7:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:7:
   GCGTAGATCT GGTCTCACCT CGCCATGGTT CG 32

(2) INFORMATION FOR SEQ ID NO:8:

(i) SEQUENCE CHARACTERISTICS:
(A) LENGTH: 61 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:8:

GCGTGGTACC TTACTCCATG GGCATGTATT CCTCTTCCTC ATCAGTCTTG TTTGTGCCTG C 61

SEQUENCE LISTING

<110> CHIRON CORPORATION

<120> A METHOD FOR GENERATION OF ANTIBODIES TO CELL SURFACE MOLECULES

<130> P044075EP

<140> 06076491.7
<141> 1993-07-08

<150> US07/910222
<151> 1992-07-09

<150> US08/015147
<151> 1993-02-09

<150> US08/070158
<151> 1993-05-28

<160> 8

<170> SeqWin99, version 1.02

<210> 1
<211> 27
<212> DNA
<213> Artificial Sequence

<220>
<223> primer

<400> 1
gcgctgcagc atctgaagcc atgggcc 27

<210> 2
<211> 27
<212> DNA
<213> Artificial Sequence

<220>
<223> primer

<400> 2
cgcggtacct tgcttctgcg gacactg 27

<210> 3
<211> 27
<212> DNA
<213> Artificial Sequence

<220>
<223> primer

<400> 3
gcgctgcagc atctgaagcc atgggcc 27

<210> 4
<211> 61
<212> DNA
<213> Artificial Sequence

<220>
<223> primer

<400> 4
gcgcggtacc ttactccatg ggcatgtatt cctcttcctc gttatcagga aaatgctgtt 60
g 61

<210>   5
<211>   32
<212>   DNA
<213>   Artificial Sequence

<220>
<223>   primer

<400>   5
gcgtagatct ggtctcacct cgccatggtt cg                                    32

<210>   6
<211>   34
<212>   DNA
<213>   Artificial Sequence

<220>
<223>   primer

<400>   6
gcgtggtacc ccacactcct gggtgggtgc agcc                                  34

<210>   7
<211>   32
<212>   DNA
<213>   Artificial Sequence

<220>
<223>   primer

<400>   7
gcgtagatct ggtctcacct cgccatggtt cg                                    32

<210>   8
<211>   61
<212>   DNA
<213>   Artificial Sequence

<220>
<223>   primer

<400>   8
gcgtggtacc ttactccatg ggcatgtatt cctcttcctc atcagtcttg tttgtgcctg   60
c                                                                      61